(19) Europäisches Patentamt / European Patent Office / Office européen des brevets

(11) **EP 3 865 709 B1**

## (12) EUROPÄISCHE PATENTSCHRIFT

(45) Veröffentlichungstag und Bekanntmachung des Hinweises auf die Patenterteilung:
**22.11.2023 Patentblatt 2023/47**

(21) Anmeldenummer: **21150377.6**

(22) Anmeldetag: **06.01.2021**

(51) Internationale Patentklassifikation (IPC):
$F04D\ 13/14^{(2006.01)}$  $F04D\ 13/06^{(2006.01)}$
$F04D\ 29/048^{(2006.01)}$  $F04D\ 29/42^{(2006.01)}$
$F04D\ 29/62^{(2006.01)}$  $A61M\ 60/113^{(2021.01)}$
$A61M\ 60/226^{(2021.01)}$  $A61M\ 60/232^{(2021.01)}$
$A61M\ 60/38^{(2021.01)}$  $A61M\ 60/422^{(2021.01)}$
$A61M\ 60/538^{(2021.01)}$  $A61M\ 60/845^{(2021.01)}$
$A61M\ 60/849^{(2021.01)}$  $F16C\ 32/04^{(2006.01)}$
$H02K\ 1/27^{(2022.01)}$  $H02K\ 5/128^{(2006.01)}$
$H02K\ 7/09^{(2006.01)}$  $H02K\ 7/14^{(2006.01)}$
$H02K\ 11/33^{(2016.01)}$  $F16C\ 41/00^{(2006.01)}$
$H02K\ 5/02^{(2006.01)}$  $H02K\ 11/20^{(2016.01)}$

(52) Gemeinsame Patentklassifikation (CPC):
F04D 13/0666; A61M 60/113; A61M 60/226;
A61M 60/232; A61M 60/38; A61M 60/422;
A61M 60/538; A61M 60/845; A61M 60/849;
F04D 13/0606; F04D 13/14; F04D 29/048;
F04D 29/426; F04D 29/628; F16C 32/0497;

(Forts.)

---

(54) **PUMPVORRICHTUNG, EINMALVORRICHTUNG UND VERFAHREN ZUM BETREIBEN EINER PUMPVORRICHTUNG**

PUMP DEVICE, DISPOSABLE DEVICE AND METHOD FOR OPERATING A PUMP DEVICE

DISPOSITIF DE POMPE, DISPOSITIF JETABLE ET PROCÉDÉ DE FONCTIONNEMENT D'UN DISPOSITIF DE POMPE

---

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.02.2020 EP 20157146**

(43) Veröffentlichungstag der Anmeldung:
**18.08.2021 Patentblatt 2021/33**

(73) Patentinhaber: **Levitronix GmbH**
**8005 Zürich (CH)**

(72) Erfinder:
• **Schöb, Reto, Dr.**
**8832 Wollerau (CH)**

• **Barletta, Natale Dr.**
**8048 Zürich (CH)**

(74) Vertreter: **IPS Irsch AG**
**Langfeldstrasse 88**
**8500 Frauenfeld (CH)**

(56) Entgegenhaltungen:
US-A- 5 405 251    US-A1- 2015 252 808
US-A1- 2017 040 868    US-A1- 2018 193 542

---

(52) Gemeinsame Patentklassifikation (CPC): (Forts.)
**H02K 1/276; H02K 5/128; H02K 7/09; H02K 7/14;
H02K 11/33;** A61M 2205/17; F16C 41/008;
H02K 5/02; H02K 11/20; H02K 2211/03;
H02K 2213/03

**Beschreibung**

Die Erfindung betrifft eine Pumpvorrichtung zum Fördern eines Fluids, eine Einmalvorrichtung für eine solche Pumpvorrichtung sowie ein Verfahren zum Betreiben einer Pumpvorrichtung.

In biotechnologischen oder medizinaltechnischen Anwendungen werden häufig Pumpen benötigt, mit denen sehr empfindliche Substanzen, wie beispielsweise Blut oder Zellkulturen oder Proteine gefördert werden können, wobei es sehr wichtig ist, dass diese Substanzen möglichst wenig durch die Pumpe geschädigt werden. Hierzu sind einerseits peristaltische Pumpen bekannt und andererseits Zentrifugalpumpen, bei welchen ein rotierender Rotor (Laufrad) mit Flügeln auf das zu fördernde Fluid einwirkt.

Es sind Zentrifugalpumpen bekannt, welche einen elektromagnetischen Drehantrieb umfassen, der nach dem Prinzip des lagerlosen Motors ausgestaltet ist und betrieben wird. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt.

Der lagerlose Motor ist dem Fachmann mittlerweile hinlänglich bekannt, und wird für zahlreiche verschiedene Anwendungen eingesetzt. Grundlegende Beschreibungen finden sich beispielsweise in der EP A 0 860 046, in der EP-A-0819330 und in der US2017/040868.

Zentrifugalpumpen, die nach dem Prinzip des lagerlosen Motors ausgestaltet sind, haben sich in einer Vielzahl von Anwendungen bewährt.

Aufgrund der Abwesenheit von mechanischen Lagern eignet sich eine Zentrifugalpumpe, die nach dem Prinzip des lagerlosen Motors ausgestaltet ist, insbesondere für solche Anwendungen, bei denen die eingangs erwähnten sehr empfindlichen Substanzen gefördert werden, beispielsweise als Blutpumpen, oder als Pumpen, bei denen sehr hohe Anforderungen an die Reinheit gestellt werden, beispielsweise in der pharmazeutischen Industrie oder in der biotechnologischen Industrie, oder auch für Anwendungen, bei denen abrasive oder aggressive Substanzen gefördert werden, welche mechanische Lager sehr schnell zerstören würden, beispielsweise Pumpen für Slurry oder säurehaltige Fluide in der Halbleiterindustrie.

Beispiele für solche Anwendungen sind die extrakorporale Membranoxygenierung (ECMO, extracorporeal membrane oxygenation), bei welcher Blut kontinuierlich durch einen Membrane-Oxygenator gefördert wird, welcher den Gasaustausch in der Lunge ersetzt, Kohlendioxid aus dem Blut entfernt und das Blut mit Sauerstoff anreichert. Ferner gibt es auch solche Maschinen, die als Herz-Lungen-Maschine (CPB, Cardiopulmonary Bypass) bei Operationen die Funktion der Lunge übernehmen. In der Biotechnologie werden Pumpen beispielsweise dafür benötigt, Nährflüssigkeiten durch einen Bioreaktor zu zirkulieren oder Fluide durch Filtereinheiten zu bewegen, wobei in den Filtereinheiten das zu generierende Substrat extrahiert wird.

Bei diesen Anwendungen, insbesondere bei den medizinaltechnischen und den biotechnologischen Anwendungen, muss natürlich eine Redundanz vorgesehen sein, denn beim Ausfall der Pumpvorrichtung muss natürlich gewährleistet sein, dass der Patient oder die zu kreierende Substanz nicht gefährdet werden. Daher ist es die gängige Praxis, eine zweite, meist identische Pumpvorrichtung bereit zu halten, welche die defekte Pumpvorrichtung ersetzt und deren Funktion übernimmt, falls diese ausfällt.

Bei allen diesen Anwendungen haben sich Zentrifugalpumpen bewährt, die nach dem Prinzip des lagerlosen Motors ausgestaltet sind, insbesondere, weil hier keine mechanischen Lager vorgesehen sind, welche einen negativen Einfluss auf die Reinheit des Prozesses haben können.

Ein weiterer Vorteil des Prinzips des lagerlosen Motors ergibt sich bei der Ausgestaltung des Rotors als Integralrotor, der sowohl der Rotor des elektromagnetischen Antriebs ist, als auch der Rotor der Zentrifugalpumpe. Neben der berührungslosen magnetischen Lagerung resultiert hier der Vorteil einer sehr kompakten und platzsparenden Ausgestaltung.

Zudem erlaubt das Prinzip des lagerlosen Motors auch Ausgestaltungen von Zentrifugalpumpen, bei denen der Rotor oder das Pumpengehäuse mit dem darin angeordneten Rotor sehr leicht vom Stator trennbar ist. Dies ist ein sehr grosser Vorteil, weil damit beispielsweise das Pumpengehäuse mit dem darin angeordneten Rotor als Einmalteil für den Einmalgebrauch ausgestaltet werden kann. Solche Einmalanwendungen ersetzen heute häufig Prozesse, bei denen früher aufgrund der sehr hohen Reinheitsanforderungen alle diejenigen Komponenten, welche im Prozess mit den zu behandelnden Fluiden in Kontakt kommen, aufwändig gereinigt und sterilisiert werden müssen, beispielsweise mittels Dampfsterilisierung. Bei der Ausgestaltung für den Einmalgebrauch werden diejenigen Komponenten, welche mit den zu behandelnden Fluiden in Kontakt kommen, nur genau einmal verwendet und dann bei der nächsten Anwendung durch neue, das heisst ungebrauchte, Einmalteile ersetzt.

Ein Problem, welches bei der Verwendung von Zentrifugalpumpen für das Fördern solcher empfindlichen Substanzen wie Blut oder anderer biologischer Fluide resultiert, ist die Wechselwirkung zwischen den Flügeln des Rotors und den zu fördernden Fluiden. Hierbei sind es insbesondere zwei Aspekte, welche die Schädigung der in dem Fluid vorhandenen Zellen, beispielsweise der roten Blutkörperchen, verursachen, nämlich die auf die Zellen oder sonstige Partikel einwirkende Scherkraft und die Verweilzeit der Scherkraft, dass heisst die Zeitdauer, über welche die Partikel dieser Scherkraft ausgesetzt sind. Mittlerweile ist es bekannt, dass die Grösse der Scherkraft der bedeutendere Faktor im Hinblick auf die Schädigung in dem geförderten Fluid darstellt.

Ausgehend von diesem Stand der Technik ist es daher eine Aufgabe der Erfindung, eine Pumpvorrichtung vorzuschlagen, welche zum einen das Fördern sehr empfindlicher Fluide, wie beispielsweise Zellen enthaltende Fluide, mit möglichst geringer Schädigung der Fluide ermöglicht und zum anderen eine Redundanz aufweist. Ferner soll ein Einmalteil für eine solche Pumpvorrichtung vorgeschlagen werden, sowie ein Verfahren zum Betreiben einer solchen Pumpvorrichtung.

Die diese Aufgabe lösenden Gegenstände der Erfindung sind durch die Merkmale des unabhängigen Patentanspruchs der jeweiligen Kategorie gekennzeichnet.

Erfindungsgemäss wird also eine Pumpvorrichtung zum Fördern eines Fluids vorgeschlagen, mit einer Einmalvorrichtung, die für den Einmalgebrauch ausgestaltet ist, und mit einer wiederverwendbaren Vorrichtung, die für den Mehrfachgebrauch ausgestaltet ist, wobei die Einmalvorrichtung zum Einsetzen in die wiederverwendbare Vorrichtung ausgestaltet ist, und zwei Pumpeneinheiten umfasst, welche in Serie hintereinander angeordnet sind, wobei jede Pumpeneinheit einen Rotor zum Fördern des Fluids umfasst, wobei jeder Rotor als Rotor eines lagerlosen Motors ausgestaltet ist, und berührungslos magnetisch lagerbar sowie berührungslos zur Rotation um eine axiale Richtung antreibbar ist, wobei die wiederverwendbare Vorrichtung zum Einsetzen der Einmalvorrichtung ausgestaltet ist, und für jeden Rotor einem Stator umfasst, der mit dem Rotor einen elektromagnetischen Drehantrieb zum Rotieren des Rotors um die axiale Richtung bildet, wobei jeder Stator als Lager- und Antriebsstator ausgestaltet ist, mit welchem der Rotor berührungslos magnetisch antreibbar und berührungslos magnetisch bezüglich des Stators lagerbar ist, und wobei für jeden Stator eine unabhängige Kontrolleinrichtung vorgesehen ist, welche für eine unabhängige Ansteuerung des jeweiligen Stators ausgestaltet ist.

Die wiederverwendbare Vorrichtung, die für den mehrmaligen Gebrauch ausgestaltet ist, ist also derart ausgebildet, dass die Einmalvorrichtung in die wiederverwendbare Vorrichtung einsetzbar ist, was ein sehr einfaches Zusammenfügen und Trennen der wiederverwendbaren Vorrichtung und der Einmalvorrichtung ermöglicht. Da die erfindungsgemässe Pumpvorrichtung zwei Pumpeneinheiten umfasst, die in Serie angeordnet sind, können für einen vorgegeben Druck, der von der Pumpvorrichtung zu generieren ist, die beiden Rotoren mit einer geringeren Drehzahl betrieben werden als wenn der gleiche Druck mit nur einer Pumpeneinheit, d.h. mit nur einem Rotor, generiert werden muss. Es ist bekannt, dass die Schädigung von Zellen - beispielsweise von roten Blutkörperchen - die in einem biologischen Fluid enthalten sind, welches mit einer Zentrifugalpumpe gefördert wird, überproportional mit der Drehzahl der Zentrifugalpumpe zunimmt. Die Drehzahl, mit welcher die Zentrifugalpumpe betrieben wird, ist der entscheidende Faktor für die Grösse der Scherkraft, denen die biologischen Zellen in dem Fluid ausgesetzt sind. Die Grösse dieser Scherkraft wiederum ist der wesentliche Faktor, welcher für die Schädigung bzw. die Zerstörung der Zellen verantwortlich ist.

Es ist bekannt, dass bei einer Zentrifugalpumpe der generierte Druck, genauer gesagt die Druckdifferenz zwischen dem Druck am Einlass der Pumpe und dem Druck am Auslass der Pumpe, zumindest in sehr guter Näherung proportional zum Quadrat der Drehzahl ist. Das bedeutet aber, wenn eine Druckdifferenz mit zwei gleichen hintereinander geschalteten Pumpeneinheiten generiert wird, so kann die Drehzahl für jede Pumpeneinheit um einen Faktor eins durch Wurzel aus zwei $\left(\frac{1}{\sqrt{2}}\right)$ gesenkt werden, das heisst, jede der beiden Pumpeneinheiten wird mit einer Drehzahl betrieben, die etwa dem 0.71-fachen der Drehzahl beträgt, mit der eine einzige Pumpeneinheit betrieben werden müsste, falls sie die gleiche Druckdifferenz generieren soll. Diese Reduzierung der Drehzahl reduziert in ganz erheblichem Masse die Schädigung von Zellen in dem zu fördernden Fluid.

Da zudem für jeden Stator eine unabhängige Kontrollvorrichtung vorgesehen ist, sodass jeder Stator unabhängig angesteuert werden kann, ermöglicht die erfindungsgemässe Pumpvorrichtung eine heisse Redundanz, sodass keine Ersatzpumpvorrichtung bereitgehalten werden muss. Im normalen, d.h. störungsfreien Betrieb, generieren die beiden hintereinandergeschalteten Pumpeneinheiten gemeinsam die gewünschte Druckdifferenz. Kommt es nun zu einem Ausfall einer der beiden Pumpeneinheiten, so wird bei der anderen Pumpeneinheit die Drehzahl um einen Faktor Wurzel zwei ($\sqrt{2}$), also etwa um einen Faktor 1.41 erhöht, sodass nun die gewünschte Druckdifferenz nur noch von dieser anderen Pumpeneinheit alleine generiert wird. Diese heisse Redundanz, welche unabhängige Ansteuerung jedes Stators ermöglicht wird, stellt insbesondere bei medizinischen Anwendungen wie dem Pumpen von Blut und biotechnologischen Anwendungen wie dem Züchten von Zellen einen ganz erheblichen Vorteil dar.

Gemäss einer bevorzugten Ausgestaltung weist die Einmalvorrichtung zwei becherförmige Ausstülpungen auf, in welchen jeweils ein Rotor vorgesehen ist, und die wiederverwendbare Vorrichtung weist zwei Ausnehmungen auf, von denen jede zur Aufnahme einer der becherförmigen Ausstülpungen ausgestaltet ist. Diese Ausgestaltung ermöglicht ein besonders einfaches Zusammensetzen und Trennen der Einmalvorrichtung und der wiederverwendbaren Vorrichtung.

Eine bevorzugte Ausführung besteht darin, dass die wiederverwendbare Vorrichtung derart ausgestaltet ist, dass die Rotoren der Einmalvorrichtung im Betriebszustand jeweils um eine Drehachse rotieren, welche mit der Vertikalen einen von Null verschiedenen Winkel einschliesst, der kleiner als 90° ist. Bei dieser Ausführung sind dann die beiden Pumpeneinheiten im Betriebszustand hintereinander in einer Ebene angeordnet, die schräg zur Horizontalen und schräg zur Vertikalen liegt. Diese Ausführung ist insbesondere für das Vorfüllen (Priming) der Pumpvorrichtung vorteilhaft, denn es kann wirkungsvoller verhindert werden, dass sich Gasblasen, beispielsweise in der Strömungsverbindung zwischen den beiden Pumpeneinheiten wie in einem Siphon festsetzen.

Gemäss einer bevorzugten Ausgestaltung ist jede Pumpeneinheit als eine radiale Pumpeneinheit konfiguriert. Die erste Pumpeneinheit weist einen ersten Einlass sowie einen ersten Auslass für das Fluid auf, die zweite Pumpeneinheit weist einen zweiten Einlass sowie einen zweiten Auslass für das Fluid auf, wobei jeder Einlass so ausgestaltet ist, dass das Fluid den jeweiligen Rotor aus einer axialen Richtung anströmt, und wobei jeder Auslass so ausgestaltet ist, dass das Fluid die jeweilige Pumpeneinheit in einer Austrittsrichtung verlässt, welche senkrecht zur axialen Richtung ausgerichtet ist.

In einem ersten Ausführungsbeispiel ist die Einmalvorrichtung der Pumpvorrichtung derart ausgestaltet, dass das Fluid zwischen dem ersten Auslass und dem zweiten Einlass um mindestens 90° und vorzugsweise um 90° umgelenkt wird. Richtet man diese Einmalvorrichtung derart aus, dass die Drehachsen der Pumpeneinheiten in vertikaler Richtung (Richtung der Schwerkraft) ausgerichtet sind, so sind die beiden Pumpeneinheiten bezüglich der axialen Richtung versetzt angeordnet. Das Fluid verlässt die erste Pumpeneinheit in einer Austrittsrichtung, die senkrecht zur vertikalen Richtung verläuft, wird dann um 90° umgelenkt und strömt dann in vertikaler Richtung durch den zweiten Einlass der zweiten Pumpeneinheit.

In einem zweiten Ausführungsbeispiel ist die Einmalvorrichtung der Pumpvorrichtung derart ausgestaltet, dass das Fluid zwischen dem ersten Auslass und dem zweiten Einlass insgesamt um 270° umgelenkt wird. Richtet man diese Einmalvorrichtung derart aus, dass die Drehachsen der Pumpeneinheiten in vertikaler Richtung (Richtung der Schwerkraft) ausgerichtet sind, so sind die beiden Pumpeneinheiten bezüglich der vertikalen Richtung auf gleicher Höhe, also nebeneinander, angeordnet. Das Fluid verlässt die erste Pumpeneinheit in einer Austrittsrichtung, die senkrecht zur vertikalen Richtung verläuft, wird dann zunächst um 90° in die vertikale Richtung nach oben umgelenkt, anschliessend wieder um 90° in eine zur vertikalen Richtung senkrechte Richtung (horizontale Richtung), dann um 90° in die vertikale Richtung nach unten umgelenkt, und strömt schliesslich in vertikaler Richtung durch den zweiten Einlass der zweiten Pumpeneinheit.

Eine vorteilhafte Massnahme besteht darin, dass für jeden Stator jeweils eine separate Energieversorgung vorgesehen ist, sodass beim Ausfall einer der Energieversorgungen noch einer der Statoren mit Energie versorgbar ist. Somit ist jeder Stator sowie die ihm zugeordnete Kontrolleinrichtung und gegebenenfalls andere Komponenten, welche diesem Stator zugeordnet sind, auch von der Energieversorgung vollkommen autark, kann also vollständig unabhängig von dem Zustand des anderen Stators bzw. der anderen Statoren nicht nur angesteuert, sondern auch mit Energie versorgt werden.

Als eine vorteilhafte Option kann eine übergeordnete Kontrolleinheit vorgesehen sein, welche mit allen Kontrolleinrichtungen für die Statoren signalverbunden ist.

Eine weitere vorteilhafte Option ist es, dass ein Notfallenergiespeicher vorgesehen ist, aus welchem jedem Stator Energie zuführbar ist, falls eine Primärenergiequelle zur Versorgung der Statoren einer oder allen Pumpeneinheiten keine Energie mehr zur Verfügung stellen kann.

Die Primärenergiequelle kann beispielsweise als Netzteil ausgestaltet sein, welches elektrischen Strom aus einem externen Stromnetz bezieht. Der Notfallenergiespeicher kann beispielsweise als ein Akkumulator oder als eine Batterie ausgestaltet sein, und stellt auch dann Energie bereit, wenn das Netzteil nicht mit einem externen Stromnetz verbunden ist, oder wenn das Netzteil defekt ist. Dieser Akkumulator bzw. diese Batterie sind beispielsweise dann sehr vorteilhaft, wenn ein Patient an die Pumpvorrichtung angeschlossen ist und von einem Ort zu einem anderen Ort transportiert werden muss.

Vorzugsweise umfasst jede Energieversorgung jeweils sowohl eine Primärenergiequelle, die als Netzteil ausgestaltet ist, das an ein externes Stromnetz angeschlossen werden kann, als auch einen Akkumulator oder eine Batterie als Notfallenergiespeicher.

Eine weitere vorteilhafte Massnahme besteht darin, dass die Einmalvorrichtung und/oder die wiederverwendbare Vorrichtung ein Identifizierungselement enthalten, mit welchem die Einmalvorrichtung und die wiederverwendbare Vorrichtung Informationen miteinander austauschen können. Jedes Identifizierungselement kann insbesondere auch als RFID (radio-frequency identification) ausgestaltet sein oder als Barcode, insbesondere als zweidimensionaler oder dreidimensionaler Barcode.

Durch die Erfindung wird ferner eine Einmalvorrichtung vorgeschlagen, die für den Einmalgebrauch und für eine Pumpvorrichtung ausgestaltet ist, die gemäss der Erfindung ausgestaltet ist.

Auch wird durch die Erfindung ein Verfahren zum Betreiben einer Pumpvorrichtung, vorgeschlagen, die gemäss der Erfindung ausgestaltet, wobei ein Soll-Wert für eine Betriebsgrösse der Pumpvorrichtung an jede Kontrolleinrichtung vorgegeben wird, wobei mittels eines Sensors ein Ist-Wert für diese Betriebsgrösse ermittelt wird, und wobei der Ist-Wert an jede Kontrolleinrichtung übermittelt wird.

Vorzugsweise tauschen die Kontrolleinrichtungen Signale miteinander aus, mittels welchen jede Kontrolleinrichtung das Funktionieren der anderen Pumpeneinheit ermitteln kann.

Eine bevorzugte Betriebsgrösse ist der Fluss durch die Pumpvorrichtung oder die von der Pumpvorrichtung generierte Druckdifferenz.

Eine vorteilhafte Option besteht darin, dass der Fluss durch die Pumpvorrichtung aus der Drehzahl und dem Drehmoment ermittelt wird, mit welchen die Rotoren angetrieben werden

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung sowohl in apparativer als auch in verfahrenstechnischer Sicht näher erläutert. In der schematischen Zeichnung zeigen (teilweise im Schnitt):

Fig. 1:      eine schematische Schnittdarstellung eines ersten Ausführungsbeispiels einer erfindungsgemässen Pumpvorrichtung in einem Schnitt entlang der Schnittlinie I-I in Fig. 2,

Fig. 2:      eine schematische Schnittdarstellung des ersten Ausführungsbeispiels in einem Schnitt entlang der Schnittlinie II-II in Fig. 1,

Fig. 3:      eine schematische Schnittdarstellung der ersten Variante für die Einmalvorrichtung in einem Schnitt entlang der Schnittlinie III-III in Fig. 4,

Fig. 4:      eine schematische Schnittdarstellung der ersten Variante für die Einmalvorrichtung in einem Schnitt entlang der Schnittlinie IV-IV in Fig. 3,

Fig. 5:      eine schematische Schnittdarstellung eines zweiten Ausführungsbeispiels einer erfindungsgemässen Pumpvorrichtung in einem Schnitt entlang der Schnittlinie V-V in Fig. 6,

Fig. 6:      eine schematische Schnittdarstellung des zweiten Ausführungsbeispiels in einem Schnitt entlang der Schnittlinie VI-VI in Fig. 5,

Fig. 7.:      eine schematische Schnittdarstellung der zweiten Variante für die Einmalvorrichtung in einem Schnitt entlang der Schnittlinie VII-VII in Fig. 8,

Fig. 8:      eine schematische Schnittdarstellung der zweiten Variante für die Einmalvorrichtung in einem Schnitt entlang der Schnittlinie VIII-VIII in Fig. 7,

Fig. 9:      eine schematische Schnittdarstellung eines dritten Ausführungsbeispiels einer erfindungsgemässen Pumpvorrichtung in einem zu Fig. 5 analogen Schnitt.

Fig. 10      eine schematische Schnittdarstellung eines vierten Ausführungsbeispiels einer erfindungsgemässen Pumpvorrichtung in einem Schnitt entlang der Schnittlinie X-X in Fig. 11,

Fig. 11:      eine schematische Schnittdarstellung des vierten Ausführungsbeispiels einer erfindungsgemässen Pumpvorrichtung in einem Schnitt entlang der Schnittlinie XI-XI in Fig. 10,

Fig. 12:      eine schematische Schnittdarstellung der dritten Variante für die Einmalvorrichtung in einem Schnitt entlang der Schnittlinie XII-XII in Fig. 13,

Fig. 13:      eine schematische Schnittdarstellung der dritten Variante für die Einmalvorrichtung in einem Schnitt entlang der Schnittlinie XIII-XIII in Fig. 12,

Fig. 14:      eine schematische Schnittdarstellung der dritten Variante für die Einmalvorrichtung in einem Schnitt entlang der Schnittlinie XIV-XIV in Fig. 12,

Fig. 15:      eine symbolische Darstellung einer Ausgestaltung einer erfindungsgemässen Pumpvorrichtung zur Erläuterung einer Ausführungsform eines erfindungsgemässen Verfahrens zum Betreiben einer erfindungsgemässen Pumpvorrichtung,

Fig. 16:     ein Flussdiagramm für die Ausführungsform des erfindungsgemässen Verfahrens, und

Fig. 17-20:  verschiedene Varianten für die Ausführungsform des erfindungsgemässen Verfahrens, jeweils in einer zu Fig. 15 analogen Darstellung.

Bei der folgenden Beschreibung der Erfindung anhand von Ausführungsbeispielen, Ausführungsformen und Varianten werden gleiche Teile oder von der Funktion her gleichwertige Teile durchweg mit den gleichen Bezugszeichen bezeichnet. Es versteht sich, dass die Erläuterungen bezüglich eines spezifischen Ausführungsbeispiels oder einer spezifischen Ausführungsform oder einer spezifischen Variante in gleicher Weise oder in sinngemäss gleicher Weise auch für die jeweils anderen Ausführungsbeispiele, Ausführungsformen und Varianten gelten. Es wird also jeweils nur auf die Unterschiede zu den vorangehend beschriebenen Ausführungsbeispielen, Ausführungsformen oder Varianten detaillierter eingegangen.

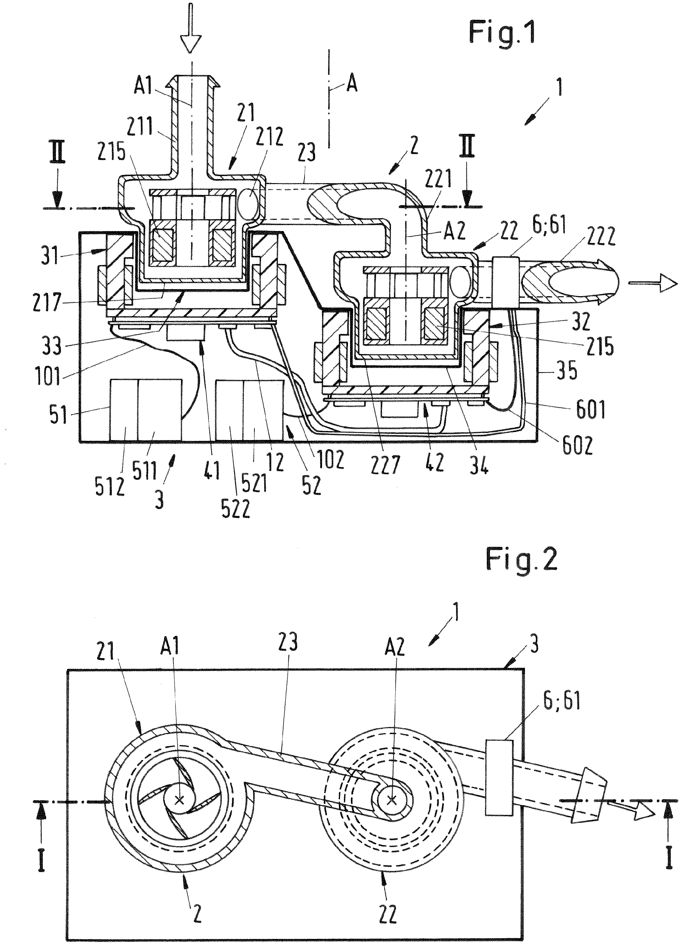

Fig. 1 zeigt in einer schematischen Schnittdarstellung ein erstes Ausführungsbeispiel einer erfindungsgemässen Pumpvorrichtung, die gesamthaft mit dem Bezugszeichen 1 bezeichnet ist. Zum besseren Verständnis zeigt Fig. 2 noch eine schematische Schnittdarstellung des ersten Ausführungsbeispiels der erfindungsgemässen Pumpvorrichtung 1, wobei der Schnitt entlang der Schnittlinie II-II in Fig. 1 erfolgt. In Fig. 2 ist die Schnittlinie I-I für die in Fig. 1 gezeigte Schnittdarstellung eingezeichnet.

Um die Reinheit bzw. die Sterilität derjenigen Komponenten zu gewährleisten, die mit dem zu fördernden Fluid, beispielsweise Blut oder ein anderes biologisches Fluid, in Kontakt kommen, weist die Pumpvorrichtung 1 eine Einmalvorrichtung auf, die gesamthaft mit dem Bezugszeichen 2 bezeichnet ist und für den Einmalgebrauch ausgestaltet ist, sowie eine wiederverwendbare Vorrichtung, die gesamthaft mit dem Bezugszeichen 3 bezeichnet ist, und die für den dauerhaften Gebrauch, also den Mehrfachgebrauch ausgestaltet ist. Dabei umfasst die Einmalvorrichtung 2 diejenigen Komponenten, welche während des Betriebs der Pumpvorrichtung 1 mit dem zu fördernden Fluid in Kontakt kommt.

Mit dem Begriff "Einmalvorrichtung" und anderen Zusammensetzungen mit dem Bestandteil "Einmal", wie z. B. Einmalteil, Einmalkomponente usw., sind dabei solche Komponenten bzw. Teile gemeint, die für den Einmalgebrauch ausgestaltet sind, die also bestimmungsgemäss nur ein einziges Mal benutzt werden können und dann entsorgt werden. Für eine neue Anwendung muss dann ein neues, bisher unbenutztes Einmalteil eingesetzt werden. Bei der Konzipierung bzw. der Ausgestaltung der Einmalvorrichtung 2 sind es daher wesentliche Aspekte, dass die Einmalvorrichtung 2 möglichst einfach und wirtschaftlich herstellbar ist, und geringe Kosten verursacht. Ein anderer wesentlicher Aspekt ist es, dass die Einmalvorrichtung 2 in möglichst einfacher Weise mit der wiederverwendbaren Vorrichtung 3 zusammenfügbar bzw. von der wiederverwendbaren Vorrichtung 3 trennbar ist. Die Einmalvorrichtung 2 soll also in sehr einfacher Weise ersetzt werden können, ohne dass dafür ein hoher Montageaufwand notwendig ist. Besonders bevorzugt soll die Einmalvorrichtung 2 ohne die Verwendung von Werkzeugen mit der wiederverwendbaren Vorrichtung 3 zusammenfügbar und von dieser trennbar sein.

Daher ist die Einmalvorrichtung 2 zum Einsetzen in die wiederverwendbare Vorrichtung 3 ausgestaltet, und die wiederverwendbare Vorrichtung 3 ist zum Aufnehmen der Einmalvorrichtung 2 ausgestaltet, d.h. derart, dass die Einmalvorrichtung 2 in die wiederverwendbare Vorrichtung 3 einsetzbar ist.

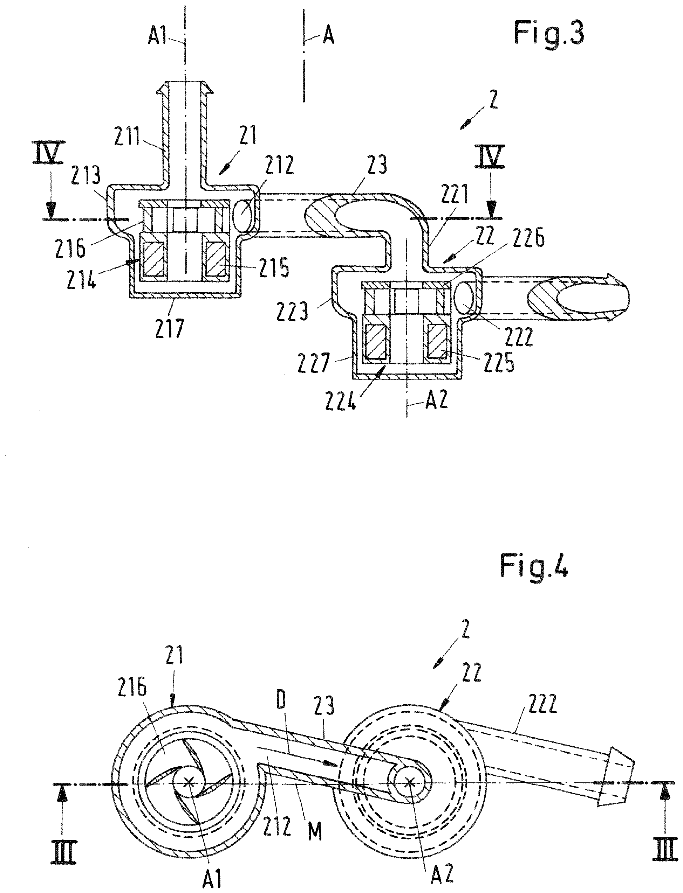

Die Einmalvorrichtung 2 ist gemäss einer ersten Variante ausgestaltet, welche in den Fig. 3 und Fig. 4 in zwei schematischen Schnittdarstellungen dargestellt ist. Dabei zeigt Fig. 3 die erste Variante der Einmalvorrichtung 2 in einem Schnitt entlang der Schnittlinie III-III in Fig. 4, und Fig. 4 zeigt die erste Variante der Einmalvorrichtung in einem Schnitt entlang der Schnittlinie IV-IV in Fig. 3.

Die Einmalvorrichtung 2 umfasst zwei Pumpeneinheiten, nämlich eine erste Pumpeneinheit 21 und eine zweite Pumpeneinheit 22. Die erste Pumpeneinheit 21 umfasst einen ersten Einlass 211 und einen ersten Auslass 212 für das zu fördernde Fluid. Die zweite Pumpeneinheit 22 umfasst einen zweiten Einlass 221 und einen zweiten Auslass 222 für das zu fördernde Fluid. Die beiden Pumpeneinheiten 21, 22 sind in Serie bzw. hintereinander angeordnet, das heisst, der erste Auslass 212 ist mittels eines Verbindungskanals 23 mit dem zweiten Einlass 221 strömungsverbunden. Im Betriebszustand strömt also das Fluid durch den ersten Einlass 211 in die erste Pumpeneinheit 21, durchströmt diese und verlässt die erste Pumpeneinheit 21 durch den ersten Auslass 212. Von dort wird das Fluid durch den Verbindungskanal 23 zum zweiten Einlass 221 geführt, durchströmt die zweite Pumpeneinheit 22 und verlässt diese durch den zweiten Auslass 222, wie dies durch die beiden Pfeile ohne Bezugszeichen in Fig. 1 dargestellt ist.

Jede Pumpeneinheit 21, 22 umfasst jeweils ein Pumpengehäuse 213 bzw. 223 (Fig. 3), in welchem jeweils ein Rotor 214 bzw. 224 zum Fördern des Fluids vorgesehen ist, welcher das jeweilige Laufrad der Pumpeneinheit 21 bildet. Jeder Rotor 214, 224 ist gleichzeitig als Rotor 214, 224 eines elektromagnetischen Drehantriebs ausgestaltet, der jeweils nach dem Prinzip eines lagerlosen Motors konfiguriert ist, was weiter hinten noch erläutert wird. Dazu umfasst jeder Rotor 214, 224 jeweils einen magnetisch wirksamen Kern 215 bzw. 225, der beispielsweise als permanentmagnetischer Ring oder auch als Ring aus einem weichmagnetischen Material, beispielsweise Eisen, gefertigt sein kann. Üblicherweise ist dieser magnetisch wirksame Kern 215 bzw. 225 vollständig ummantelt bzw. eingekapselt, wobei die Ummantelung vorzugsweise aus einem Kunststoff besteht. Auf dieser Ummantelung ist dann jeweils ein Flügelrad 216 bzw. 226 vorgesehen, welches mit einer Mehrzahl von Flügeln auf das zu fördernde Fluid einwirkt.

Vorzugsweise ist jede Pumpeneinheit 21, 22 als eine radiale Zentrifugalpumpe ausgestaltet, bei welcher der jeweilige Rotor 214, 215 um eine Drehachse A1 bzw. A2 rotiert. Der jeweilige Rotor 214, 224 wird von dem Fluid in Richtung der jeweiligen Drehachse A1 bzw. A2 angeströmt und lenkt das Fluid in eine Austrittsrichtung D (Fig. 4) um, welche senkrecht auf der jeweiligen Drehachse A1 bzw. A2 steht.

Vorzugsweise - aber nicht notwendigerweise - sind die beiden Pumpeneinheiten 21, 22 zumindest bezüglich ihrer Hydraulik identisch ausgestaltet. Besonders bevorzugt sind die Drehachsen A1 und A2 parallel zueinander. Diese gemeinsame Richtung, in welcher sich die beiden parallelen Drehachsen A1, A2 erstrecken, wird im Folgenden als axiale Richtung A bezeichnet. Eine dazu senkrechte Richtung wird als radiale Richtung bezeichnet.

Ferner ist es bevorzugt, dass die beiden Pumpeneinheiten 21, 22 durch den Verbindungskanal 23 starr miteinander verbunden, sodass die beiden Pumpeneinheiten 21, 22 mit dem Verbindungskanal 23 eine bauliche Einheit bilden, welche gesamthaft in die wiederverwendbare Vorrichtung 3 einsetzbar ist.

Die Längsrichtung des Verbindungskanals 23 bestimmt die Austrittsrichtung D (Fig. 4), in welcher das Fluid die erste Pumpeneinheit 21 verlässt. Der Verbindungskanal 23 ist so ausgerichtet, dass die Austrittsrichtung D einerseits senkrecht auf der axialen Richtung A steht, und andererseits mit einer gedachten, kürzesten Verbindungslinie M zwischen den beiden Drehachsen A1 und A2 einen von 0° und von 90° verschiedenen Winkel bildet. Die Verbindungslinie M liegt in der Darstellung in Fig. 4 auf der Schnittlinie III-III.

Wie dies insbesondere in den Fig. 1 und Fig. 3 zu erkennen ist, ist bei der ersten Variante der Einmalvorrichtung 2 der Verbindungskanal 23 so ausgestaltet, dass das Fluid bezüglich der axialen Richtung A zwischen dem ersten Auslass 212 und dem zweiten Einlass 221 um 90° umgelenkt wird. Der Verbindungskanal 23 erstreckt sich stromabwärts des ersten Auslasses 212 zunächst senkrecht zur axialen Richtung A, sodass die Austrittsrichtung D senkrecht auf der axialen Richtung A steht. Anschliessend krümmt sich der Verbindungskanal 23 um 90° nach unten, wobei sich "unten" auf die Darstellung in Fig. 1 und in Fig. 3 bezieht, sodass das Fluid den zweiten Einlass 221 in axialer Richtung A durchströmt. Diese Ausgestaltung hat zur Folge, dass in der in Fig. 1 dargestellten Gebrauchslage, in welcher die Drehachsen A1, A2 der Pumpeneinheiten 21, 22 jeweils in vertikaler Richtung (Richtung der Schwerkraft) ausgerichtet sind, die beiden Pumpeneinheiten 21, 22 bezüglich der vertikalen Richtung versetzt angeordnet sind.

Jede Pumpeneinheit 21, 22 weist jeweils eine topf- oder becherförmige Ausstülpung 217 bzw. 227 auf, die hier von dem jeweiligen Pumpengehäuse 213 bzw. 223 gebildet wird. Jeder Rotor 214, 224 ist in dem zugehörigen Pumpengehäuse 213 bzw. 223 so angeordnet, dass zumindest der magnetisch wirksame Kern 215 bzw. 225 des jeweiligen Rotors 214, 224 in der jeweiligen becherförmigen Ausstülpung 217 bzw. 227 angeordnet ist.

Die widerverwendbare Vorrichtung 3 (Fig. 1) umfasst ein Statorgehäuse 35, in welchem zwei Statoren, nämlich ein erster Stator 31 und ein zweiter Stator 32, zum Zusammenwirken mit jeweils einem der Rotoren 214 bzw. 224 vorgesehen sind. Die wiederverwendbare Vorrichtung 3 umfasst ferner zwei topf- oder becherförmige Ausnehmungen 33 bzw. 34, die in dem Statorgehäuse 35 vorgesehen sind, und welche so bemessen und angeordnet sind, dass jede der becherförmigen Ausnehmungen 33, 34 jeweils eine der becherförmigen Ausstülpungen 217, 227 der Einmalvorrichtung 2 aufnehmen und umschliessen kann. Die Abmessungen jeder Ausnehmung 33, 34 und jeder Ausstülpung 217, 227 sind dabei so aufeinander abgestimmt, dass jede Ausnehmung 33, 34 jeweils eine der Ausstülpungen 217, 227 im zusammengesetzten Zustand eng umschliesst und mit ihrer Mantelfläche an der Mantelfläche der jeweiligen Ausstülpung 217, 227 anliegt.

Die beiden Statoren 31, 32 sind jeweils um eine der Ausnehmungen 33 bzw. 34 herum angeordnet und umschliessen die jeweilige Ausnehmung 33, 34 möglichst eng. Dabei ist jeder Stator 31, 32 so angeordnet, dass im zusammengesetzten Zustand der Pumpvorrichtung 1 jeder der magnetisch wirksamen Kerne 215, 225 von jeweils einem der Statoren 31, 32 umgeben wird bzw. von den Statorpolen des Stators 31 bzw. 32 umgeben wird, sodass eine möglichst gute magnetische Wechselwirkung zwischen dem jeweiligen Stator 31, 32 und dem jeweiligen magnetisch wirksamen Kern 215, 225 gegeben ist.

Die Ausgestaltung mit den Ausstülpungen 217, 227 in der Einmalvorrichtung 2 und den Ausnehmungen 33, 34 in der wiederverwendbaren Vorrichtung 3 ermöglicht ein besonders einfaches Verbinden bzw. Trennen der Einmalvorrichtung 2 und der wiederverwendbaren Vorrichtung 3. Die beiden Ausstülpungen 217 und 227 mit den darin angeordneten Rotoren 214, 224 werden in einfacher Weise in die beiden Ausnehmungen 33, 34 eingesetzt und schon ist die Pumpvorrichtung 1 betriebsbereit. In genauso einfacher Weise können die beiden Ausstülpungen 217, 227 aus den Ausnehmungen 33, 34 herausgezogen werden, wodurch die Einmalvorrichtung 2 von der wiederverwendbaren Vorrichtung 3 getrennt wird. Selbstverständlich können Sicherungselemente wie beispielsweise eine Schnappverbindung vorgesehen sein, welche ein unbeabsichtigtes Trennen der Einmalvorrichtung 2 und der wiederverwendbaren Vorrichtung 3 vermeiden.

Wie bereits erwähnt sind die beiden Rotoren 214, 224 und die beiden Statoren 31, 32 so ausgestaltet, dass jeweils der erste Stator 31 und der erste Rotor 214 sowie der zweite Stator 32 und der zweite Rotor 224 einen elektromagnetischen Drehantrieb bilden, welcher als lagerloser Motor ausgestaltet ist.

Dazu ist jeder Stator 31, 32 jeweils als Lager- und Antriebsstator ausgestaltet, mit welchem der jeweilige Rotor 214, 224 berührungslos magnetisch zur Rotation um die jeweilige Drehachse A1, A2 antreibbar und berührungslos magnetisch bezüglich des Stators 31, 32 lagerbar ist.

Da die beiden Drehachsen A1, A2 parallel sind, wird im Folgenden auf die axiale Richtung A Bezug genommen.

Mit dem magnetisch wirksamen Kern 215 bzw. 225 des Rotors 214 bzw. 224, welcher in Form einer Kreisscheibe, beziehungsweise eines Kreiszylinders, oder ringförmig ausgestaltet sein kann, ist derjenige Bereich des Rotors 214, 224 gemeint, welcher für die Drehmomentbildung sowie für die Erzeugung der magnetischen Lagerkräfte mit dem jeweiligen Stator 31, 32 zusammenwirkt. Je nach Ausgestaltung kann der magnetisch wirksame Kern 215, 225 einen oder mehrere Permanentmagnete umfassen. Alternativ ist es auch möglich, den magnetisch wirksamen Kern 215, 225 ohne Permanentmagnete auszugestalten, beispielsweise als Reluktanzläufer. Dabei besteht der magnetisch wirksame Kern 215, 225 zumindest teilweise aus einem ferromagnetischen Material, beispielsweise Eisen.

Der jeweilige Drehantrieb mit dem ersten Rotor 214 und dem ersten Stator 31 bzw. mit dem zweiten Rotor 224 und dem zweiten Stator 32 ist beispielsweise als sogenannter Tempelmotor ausgestaltet.

Das Charakteristische einer Ausgestaltung als Tempelmotor ist es, dass der Stator 31, 32 eine Mehrzahl von separaten Spulenkernen umfasst, von denen jeder einen stabförmigen Längsschenkel umfasst, welcher sich von einem ersten Ende in axialer Richtung A bis zu einem zweiten Ende erstreckt, wobei alle ersten Enden durch einen Rückschluss miteinander verbunden sind. Ferner umfasst jeder Spulenkern einen Querschenkel, welcher an dem zweiten Ende des jeweiligen Längsschenkels angeordnet ist, und welcher sich in radialer Richtung, also senkrecht zur axialen Richtung A und damit senkrecht zu dem jeweiligen Längsschenkel erstreckt. Jeder Querschenkel erstreckt sich in radialer Richtung nach innen, also auf den jeweiligen Rotor 214, 224 zu. Jeder Spulenkern hat somit eine L-förmige Ausgestaltung, wobei die Längsschenkel jeweils den sich in axialer Richtung A erstreckenden langen Schenkel des L bilden, und die sich senkrecht zu den Längsschenkeln in radialer Richtung auf den Rotor 214, 224 hin gerichtet erstreckenden Querschenkel jeweils den kurzen Schenkel des L bilden.

Die radial innen liegenden Enden der Querschenkel bilden jeweils einen Statorpol. Die Statorpole sind ringförmig um die jeweilige Ausnehmung 33, 34 mit dem darin befindlichen Rotor 214 oder 224 herum angeordnet. Im Betriebszustand befinden sich die Statorpole und der magnetisch wirksame Kern 215 bzw. 225 bezüglich der axialen Richtung A auf gleicher Höhe, wenn der Rotor 214, 224 nicht aus seiner Solllage ausgelenkt ist.

Die zueinander parallel ausgerichteten Längsschenkel der Spulenkerne, die sich alle parallel zur axialen Richtung A erstrecken, und welche den Rotor 214 bzw. 224 umgeben, sind es, welche dem Tempelmotor seinen Namen gegeben haben, weil diese parallelen Längsschenkel an die Säulen eines Tempels erinnern.

Jeder Stator 31, 32 umfasst ferner eine Mehrzahl von Wicklungen zur Erzeugung von elektromagnetischen Drehfeldern, mit welchen der jeweilige Rotor 214, 224 berührungslos magnetisch antreibbar und berührungslos magnetisch bezüglich des Stators 31 bzw. 32 lagerbar ist. Die Wicklungen sind beispielsweise als individuelle Spulen ausgestaltet, wobei an jedem der Längsschenkel eines Stators 31, 32 jeweils eine Spule vorgesehen ist. Jede Spule ist um den jeweiligen Längsschenkel herum angeordnet, sodass die Spulenachse jeweils parallel zur axialen Richtung A liegt.

Jeder Tempelmotor ist nach dem Prinzip eines lagerlosen Motors ausgestaltet. Das heisst, während des Betriebs der Pumpvorrichtung 1 wirkt der jeweilige magnetisch wirksame Kern 215, 225 des Rotors 214, 224 mit dem jeweiligen Stators 31, 32 nach dem eingangs beschriebenen Prinzip des lagerlosen Motors zusammen, bei welchem der jeweilige Rotor 214, 224 berührungslos magnetisch antreibbar und berührungslos magnetisch bezüglich des jeweiligen Stators 31, 32 lagerbar ist.

Das Prinzip des lagerlosen Motors ist dem Fachmann mittlerweile hinlänglich bekannt, sodass eine detaillierte Beschreibung der Funktion nicht mehr notwendig ist. Mit dem Prinzip des lagerlosen Motors ist gemeint, dass der Rotor 214, 224 magnetisch antreibbar und magnetisch lagerbar ist, wobei der Stator 31, 32 als Lager- und Antriebsstator ausgestaltet ist, der sowohl Stator des elektrischen Antriebs als auch Stator der magnetischen Lagerung ist. Dazu umfasst der Stator 31 oder 32 jeweils die Wicklungen, mit denen sowohl die Antriebsfunktion als auch die Lagerfunktion realisiert wird. Mit den Wicklungen lässt sich ein elektromagnetisches Drehfeld erzeugen, welches zum einen ein Drehmoment auf den magnetisch wirksamen Kern 215, 225 des Rotors 214, 224 ausübt, das dessen Rotation um die axiale Richtung A bewirkt, und welches zum anderen eine beliebig einstellbare Querkraft auf den magnetisch wirksamen Kern 215, 225 des Rotors 214, 224 ausübt, sodass dessen radiale Position -also seine Position in der zur axialen Richtung A senkrechten radialen Ebene- aktiv steuerbar bzw. regelbar ist. Beim lagerlosen Motor wird also im Unterschied zu klassischen Magnetlagern die magnetische Lagerung und der Antrieb des Motors über elektromagnetische Drehfelder realisiert, die ein Drehmoment und eine einstellbare Querkraft auf den magnetisch wirksamen Kern des Rotors ausüben. Die hierfür benötigten Drehfelder können entweder mit unterschiedlichen Spulen generiert werden, oder die Drehfelder können durch rechnerische Überlagerung der benötigten Ströme und dann mithilfe eines einzigen Spulensystems generiert werden. Es ist also bei einem lagerlosen Motor nicht möglich, den elektromagnetischen Fluss, der von den Wicklungen des Stators 31, 32 generiert wird, aufzuteilen in einen elektromagnetischen Fluss, der nur für den Antrieb des Rotors 214, 224 sorgt, und einen elektromagnetischen Fluss, der nur die magnetische Lagerung des Rotors 214, 224 realisiert.

Gemäss dem Prinzip des lagerlosen Motors sind zumindest drei Freiheitsgrade des Rotors 214, 224, nämlich seine Position in der radialen Ebene und seine Rotation um die axiale Richtung A, aktiv regelbar. Bezüglich seiner axialen Auslenkung in axialer Richtung A ist der magnetisch wirksame Kern 215, 225 des Rotors 214, 224 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Drehachse A1, A2 senkrechten radialen Ebene, ist der Rotor 214, 224 ebenfalls passiv magnetisch stabilisiert. Der Rotor 214, 224 ist also durch das Zusammenwirken des magnetisch wirksamen Kerns 215, 225 mit dem Stator 31, 32 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert. Auf diese Weise ist der jeweilige Rotor 214, 224 berührungslos magnetisch zur Rotation um die jeweilige Drehachse A1, A2 antreibbar, und berührungslos magnetisch bezüglich des jeweiligen Stators 31, 32 lagerbar.

Ein wesentlicher Aspekt der erfindungsgemässen Pumpvorrichtung 1 ist es, dass für jeden Stator 31, 32 jeweils eine unabhängige Kontrolleinrichtung 41, 42 vorgesehen ist, nämlich eine erste Kontrolleinrichtung 41 für den ersten Stator 31 und eine zweite Kontrolleinrichtung 42 für den zweiten Stator 32. Dabei ist jede Kontrolleinrichtung 41, 42 jeweils derart ausgestaltet, dass eine unabhängige Ansteuerung des jeweiligen Stators 31, 32 möglich ist.

Prinzipiell benötigt also die erste Kontrolleinrichtung 41 keine Informationen von der zweiten Kontrolleinrichtung 42, um den ersten Stator 31 und den ersten Rotor 214 nach dem Prinzip des lagerlosen Motors zu betreiben. Umgekehrt benötigt die zweite Kontrolleinrichtung 42 keine Informationen von der ersten Kontrolleinrichtung 41, um den zweiten Stator 32 und den zweiten Rotor 224 nach dem Prinzip des lagerlosen Motors zu betreiben.

Wenn also eine der Kontrolleinrichtungen 41 oder 42 ausfällt, so ist die Pumpvorrichtung 1 nach wie vor funktionstüchtig, weil mit der anderen Kontrolleinrichtung 42 oder 41 der Betrieb der Pumpvorrichtung 1 aufrechterhalten werden kann. Somit ist die erfindungsgemässe Pumpvorrichtung 1 heiss redundant ausgestaltbar.

Vorzugsweise ist jede Kontrolleinrichtung 41 bzw. 42 jeweils als ein Elektronikprint ausgestaltet, der gemäss der Darstellung in Fig. 1 unterhalb des jeweiligen Stators 31, 32 angeordnet und an dem jeweiligen Stator 31, 32 befestigt ist. Jeder Elektronikprint umfasst alle für den Betrieb des jeweiligen lagerlosen Motors benötigten Komponenten wie beispielsweise die Leistungselektronik zum Ansteuern der Wicklungen, sowie die benötigten Auswerte-, Regel- und Ansteuerkomponenten.

Ferner ist für jede Kontrolleinrichtung 41, 42 jeweils eine Energieversorgung 51, 52 vorgesehen, welche die jeweilige Kontrolleinrichtung 41, 42 mit Energie, vorzugsweise mit elektrischer Energie versorgt, nämlich eine erste Energieversorgung 51, welche die erste Kontrolleinrichtung 41 mit Energie versorgt, und eine zweite Energieversorgung 52, welche die zweite Kontrolleinrichtung 42 mit Energie versorgt. Die erste Energieversorgung 51 ist über eine erste Versorgungsleitung 101 mit der ersten Kontrolleinrichtung 41 verbunden, und die zweite Energieversorgung 52 ist über eine zweite Versorgungsleitung 102 mit der zweiten Kontrolleinrichtung 42 verbunden.

Vorzugsweise sind die Energieversorgungen 51, 52 im Statorgehäuse 35 angeordnet.

Jede Energieversorgung 51, 52 umfasst eine Primärenergiequelle 511, 521 sowie einen Notfallenergiespeicher 512, 522. Die Primärenergiequelle 511, 521 kann beispielsweise jeweils als Netzteil ausgestaltet sein, welches elektrischen Strom aus einem externen Stromnetz bezieht. Der Notfallenergiespeicher 512, 522 kann beispielsweise jeweils als ein Akkumulator oder als eine Batterie ausgestaltet sein, und stellt auch dann Energie bereit, wenn das Netzteil nicht mit einem externen Stromnetz verbunden ist, oder wenn das Netzteil defekt ist. Dieser Akkumulator bzw. diese Batterie sind beispielsweise dann sehr vorteilhaft, wenn ein Patient an die Pumpvorrichtung 1 angeschlossen ist und von einem Ort zu einem anderen Ort transportiert werden muss.

In anderen Ausführungsformen ist nur eine gemeinsame Energieversorgung vorgesehen, die beide Kontrolleinrichtungen 41, 42 mit elektrischer Energie versorgt. Auch bei solchen Ausgestaltungen umfasst die gemeinsame Energieversorgung sowohl eine Primärenergiequelle, die als Netzteil ausgestaltet ist, das an ein externes Stromnetz angeschlossen werden kann, und einen Akkumulator oder eine Batterie als Notfallenergiespeicher.

Die Pumpvorrichtung 1 umfasst ferner mindestens einen Sensor 6, mit welchem eine Betriebsgrösse der Pumpvorrichtung 1 ermittelt werden kann. Der Sensor 6 ist beispielsweise ein Flusssensor 61, mit welchem der Fluss des Fluids durch die Pumpvorrichtung 1 bestimmbar ist. Der Flusssensor 61, der beispielsweise als Ultraschall-Durchflussmesseinrichtung ausgestaltet ist, kann am oder in der Nähe des zweiten Auslasses 222 angeordnet sein. Dabei ist es möglich, dass der Flusssensor 61, oder allgemeiner der Sensor 6, Bestandteil der wiederverwendbaren Vorrichtung 3 ist. Ferner ist es möglich, dass der Flusssensor 61, oder allgemeiner der Sensor 6, Bestandteil der Einmalvorrichtung 2 ist.

Der Sensor 6 ist mittels einer ersten Signalverbindung 601 mit der ersten Kontrolleinrichtung 41 signalverbunden und mittels einer zweiten Signalverbindung 602 mit der zweiten Kontrolleinrichtung 42 signalverbunden. Auch dadurch ist die Unabhängigkeit der Kontrolleinrichtungen 41, 42 voneinander gewährleistet, denn jede Kontrolleinrichtung 41, 42 enthält unabhängig von der jeweils anderen Kontrolleinrichtung 42, 41 das Signal von dem Sensor 6.

Vorzugsweise ist eine Kommunikationsverbindung 12 vorgesehen, über welche die beiden Kontrolleinrichtungen 41 und 42 Signale oder Informationen miteinander austauschen können. Diese Kommunikationsverbindung 12 kann beispielsweise dazu genutzt werden, dass jede Kontrolleinrichtung 41, 42 die Funktionstüchtigkeit der jeweils anderen Kontrolleinrichtung 42, 41 bzw. der jeweils anderen Pumpeneinheit 22, 21 überprüfen kann.

Die Einmalvorrichtung 2 wird - mit Ausnahme der magnetisch wirksamen Kerne 215, 225 - vorzugsweise aus einem oder mehreren Kunststoffen gefertigt. Insbesondere sind die Pumpengehäuse 213, 223, die Einlässe 211, 221, die Auslässe 212, 222, der Verbindungskanal 23, die Flügelräder 216, 226, die Ausstülpungen 217, 227 sowie die Ummantelungen der magnetisch wirksamen Kerne 215, 225 aus Kunststoff hergestellt. Selbstverständlich müssen nicht alle Komponenten der Einmalvorrichtung 2 aus dem gleichen Kunststoff gefertigt sein.

Die Auswahl geeigneter Kunststoffe richtet sich natürlich nach dem jeweiligen Anwendungsfall. Geeignete Kunststoffe sind beispielsweise: PolyEthylene (PE), PolyPropylene (PP), Low Density PolyEthylene (LDPE), Ultra Low Density PolyEthylene (ULDPE), Ethylene Vinyl Acetate (EVA), PolyEthylene Terephthalate (PET), PolyVinylChlorid (PVC), PolyVinyliDene Fluoride (PVDF), Acrylonitrile Butadiene Styrene (ABS), PolyUrethan (PU), PolyAcryl, PolyCarbonate (PC), Silicone.

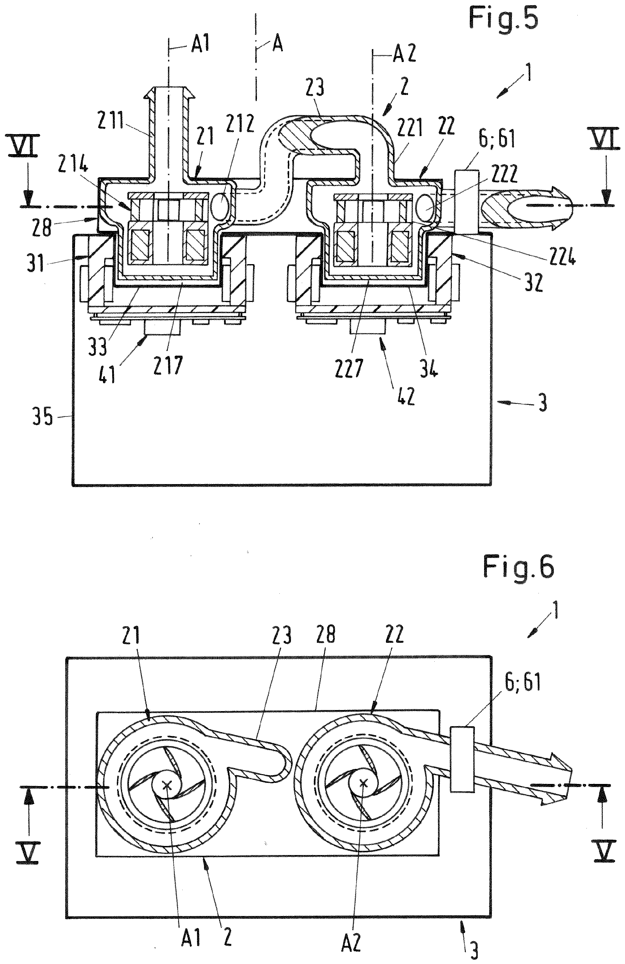

Im Folgenden wird anhand der Fig. 5 - Fig. 8 ein zweites Ausführungsbeispiel einer erfindungsgemässen Pumpvorrichtung 1 erläutert, welches eine zweite Variante für die Einmalvorrichtung 2 aufweist. Wie bereits erwähnt, wird dabei nur auf die Unterschiede zum ersten Ausführungsbeispiel und zur ersten Variante detaillierter eingegangen.

Fig. 5 zeigt in einer zu Fig. 1 analogen Darstellung eine schematische Schnittdarstellung des zweiten Ausführungsbeispiels der erfindungsgemässen Pumpvorrichtung 1 in einem Schnitt entlang der Schnittlinie V-V in Fig. 6. Allerdings ist in Fig. 5 auf die Darstellung der Energieversorgungen 51, 52 sowie der diversen Verbindungen im Statorgehäuse 35 verzichtet worden, weil diese in analoger Weise wie bei dem ersten Ausführungsbeispiel ausgestaltet sind.

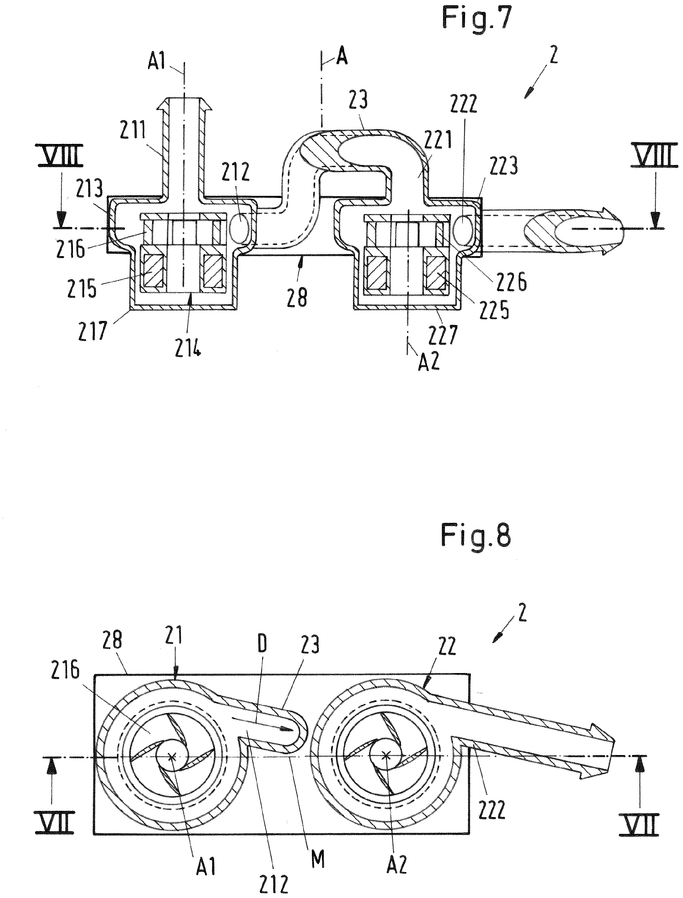

Fig. 6 zeigt in einer zu Fig. 2 analogen Darstellung eine schematische Schnittdarstellung des zweiten Ausführungsbeispiels der erfindungsgemässen Pumpvorrichtung 1 in einem Schnitt entlang der Schnittlinie VI-VI in Fig. 5. Fig. 7 zeigt in einer zu Fig. 3 analogen Darstellung eine schematische Schnittdarstellung der zweiten Variante für die Einmalvorrichtung 2 in einem Schnitt entlang der Schnittlinie VII-VII in Fig. 8. Fig. 8 zeigt in einer zu Fig. 4 analogen Darstellung eine schematische Schnittdarstellung der zweiten Variante für die Einmalvorrichtung 2 in einem Schnitt entlang der Schnittlinie VIII-VIII in Fig. 7.

Bei der zweiten Variante der Einmalvorrichtung 2 ist ein Einmalgehäuse 28 vorgesehen, in welchem die Pumpeneinheiten 21, 22 angeordnet sind. Das Einmalgehäuse 28 bildet beispielsweise eine Kassette, welche als Einmalteil in besonders einfacher Weise in die wiederverwendbare Vorrichtung 3 einsetzbar, bzw. von der wiederverwendbaren Vorrichtung 3 trennbar ist.

Wie dies insbesondere in den Fig. 5 und Fig. 7 zu erkennen ist, ist bei der zweiten Variante der Einmalvorrichtung 2 der Verbindungskanal 23 so ausgestaltet, dass das Fluid bezüglich der axialen Richtung A zwischen dem ersten Auslass 212 und dem zweiten Einlass 221 um insgesamt 270° umgelenkt wird. Der Verbindungskanal 23 ist im Wesentlichen U-förmig ausgestaltet. Der Verbindungskanal 23 erstreckt sich stromabwärts des ersten Auslasses 212 zunächst senkrecht zur axialen Richtung A, sodass die Austrittsrichtung D (Fig. 8) senkrecht auf der axialen Richtung A steht. Anschliessend krümmt sich der Verbindungskanal 23 um 90° nach oben, wobei sich "oben" auf die Darstellung in Fig. 5 und in Fig. 7 bezieht. Danach krümmt sich der Verbindungskanal 23 um 90° nach rechts, wobei sich "rechts" auf die Darstellung in Fig. 5 und in Fig. 7 bezieht, wodurch der Verbindungskanal 23 wieder senkrecht zur axialen Richtung A ausgestaltet ist. Schliesslich krümmt sich der Verbindungskanal 23 um 90° nach unten, wobei sich "unten" auf die Darstellung in Fig. 5 und in Fig. 7 bezieht, sodass das Fluid den zweiten Einlass 221 in axialer Richtung A durchströmt. Diese Ausgestaltung hat zur Folge, dass in der in Fig. 5 dargestellten Gebrauchslage, in welcher die Drehachsen A1, A2 der Pumpeneinheiten 21, 22 jeweils in vertikaler Richtung (Richtung der Schwerkraft) ausgerichtet sind, die beiden Pumpeneinheiten 21, 22 bezüglich der vertikalen Richtung auf gleicher Höhe, das heisst nebeneinander angeordnet sind.

Die wiederverwendbare Vorrichtung 3 ist an diese Ausgestaltung der Einmalvorrichtung 2 angepasst.

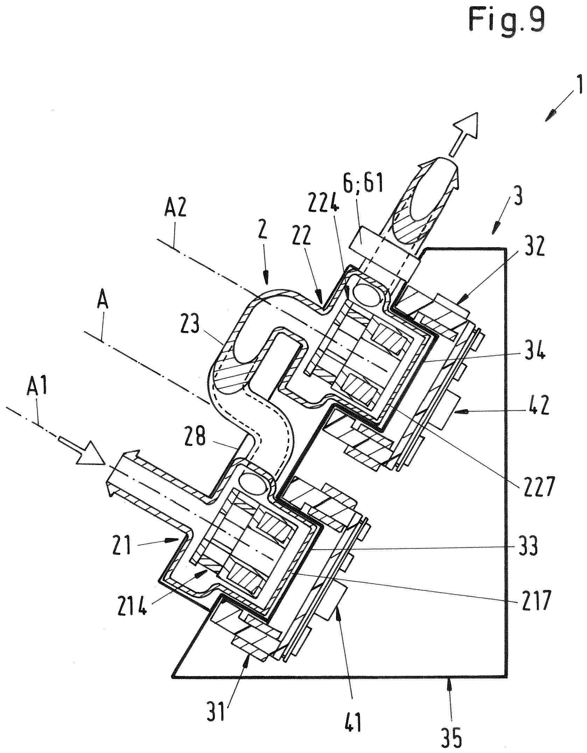

Fig. 9 zeigt eine schematische Schnittdarstellung ein drittes Ausführungsbeispiel einer erfindungsgemässen Pumpvorrichtung 1 in einem zu Fig. 5 analogen Schnitt. Das dritte Ausführungsbeispiel der erfindungsgemässen Pumpvorrichtung 1 umfasst eine Einmalvorrichtung 2, die gemäss der zweiten Variante der Einmalvorrichtung 2 ausgestaltet ist. Es versteht sich, dass das dritte Ausführungsbeispiel der erfindungsgemässen Pumpvorrichtung 1 in sinngemäss gleicher Weise auch mit einer Einmalvorrichtung 2 versehen sein kann, die gemäss der ersten Variante für die Einmalvorrichtung 2. ausgestaltet ist.

Der wesentliche Unterschied zu den vorangehend beschriebenen Ausführungsbeispielen ist es, dass bei dem dritten Ausführungsbeispiel der erfindungsgemässen Pumpvorrichtung 1 die wiederverwendbare Vorrichtung 3 derart ausgestaltet ist, dass die Rotoren 214, 224 der Einmalvorrichtung 2 im Betriebszustand jeweils um die Drehachse A1, A2 rotieren, wobei die Drehachsen A1 und A2 mit der Vertikalen einen von Null verschieden Winkel einschliessen, der kleiner als 90° ist. Das heisst, die beiden Drehachsen A1 und A2 sind im Betriebszustand unter einem von Null und 90° verschiedenen Winkel gegen die Vertikale geneigt. Die Vertikale entspricht der vertikalen Richtung, also der Richtung, in welcher die Gravitation wirkt.

Mit dieser Ausgestaltung lässt es sich besonders einfach vermeiden, dass sich beim Vorfüllen (Priming) der Pumpvorrichtung 1 insbesondere in dem Verbindungskanal 23 Gasblasen, beispielsweise Luftblasen, festsetzen, die im Betrieb zu erheblichen Schädigungen, beispielsweise eines an die Pumpvorrichtung 1 angeschlossenen Patienten führen könnten. Durch die Schrägstellung der beiden Pumpeneinheiten 21, 22 relativ zur vertikalen und zur horizontalen Richtung wird ein Festsetzen von Gasblasen, wie es beispielsweise in einem Siphon erwünscht ist, effizient unterbunden.

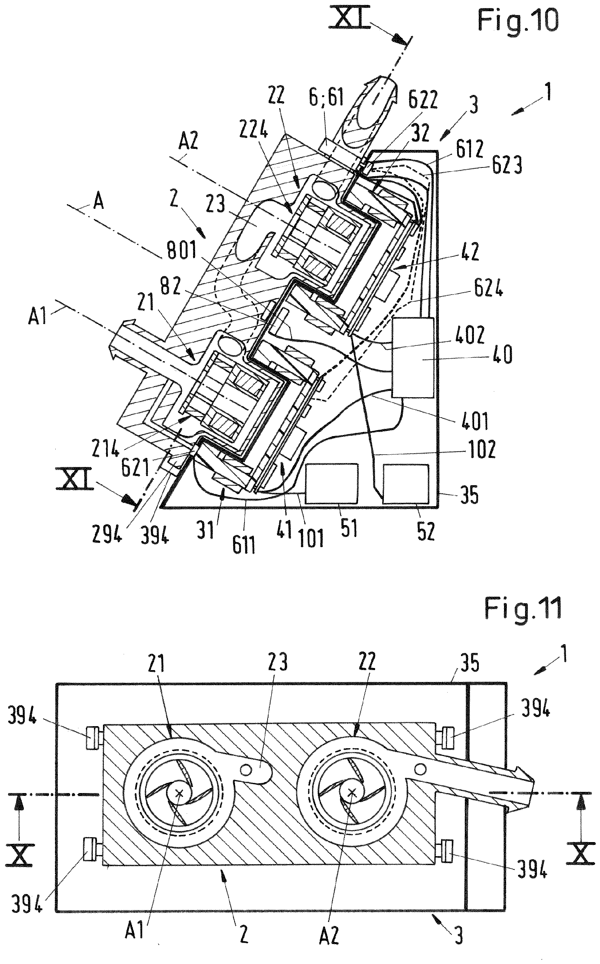

In den Fig. 10 - Fig. 14 ist ein viertes Ausführungsbeispiel einer erfindungsgemässen Pumpvorrichtung 1 erläutert, welches eine dritte Variante für die Einmalvorrichtung 2 aufweist. Auch hier wird nur auf die Unterschiede zu den vorangehend beschriebenen Ausführungsbeispielen und Varianten detaillierter eingegangen.

Es versteht sich, dass das vierte Ausführungsbeispiel der erfindungsgemässen Pumpvorrichtung 1 in sinngemäss gleicher Weise auch mit einer Einmalvorrichtung 2 versehen sein kann, die gemäss der ersten oder zweiten Variante für die Einmalvorrichtung 2. ausgestaltet ist.

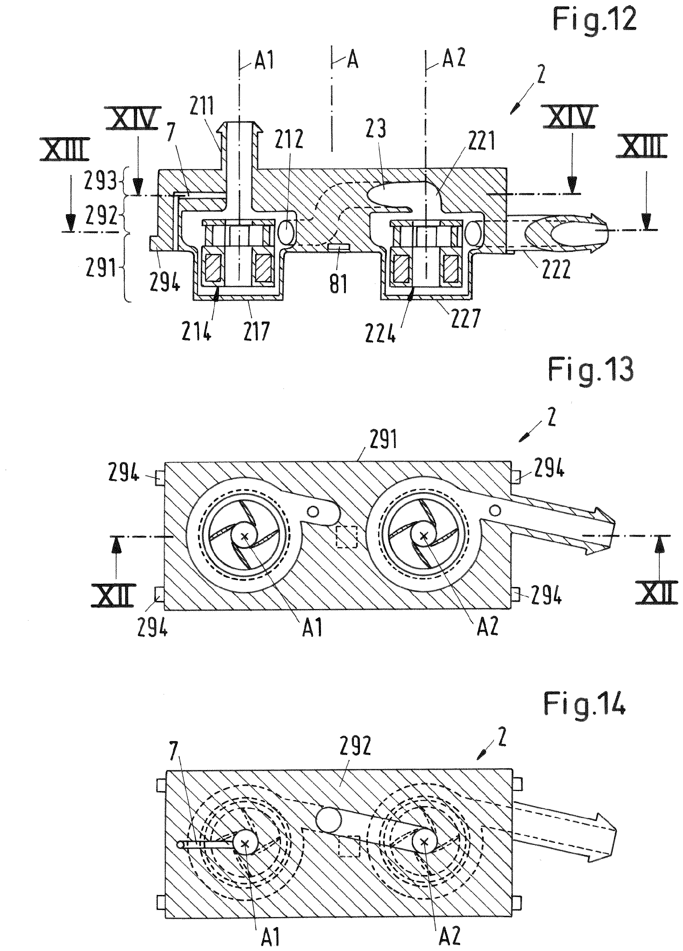

Fig. 10 zeigt in einer zu Fig. 1 analogen Darstellung eine schematische Schnittdarstellung des vierten Ausführungsbeispiels einer erfindungsgemässen Pumpvorrichtung 1 in einem Schnitt entlang der Schnittlinie X-X in Fig. 11. Fig. 11 zeigt in einer zu Fig. 6 analogen Darstellung eine schematische Schnittdarstellung des vierten Ausführungsbeispiels der erfindungsgemässen Pumpvorrichtung 1 in einem Schnitt entlang der Schnittlinie XI-XI in Fig. 10. Fig. 12 zeigt in einer zu Fig. 7 analogen Darstellung eine schematische Schnittdarstellung der dritten Variante für die Einmalvorrichtung 2 in einem Schnitt entlang der Schnittlinie XII-XII in Fig. 13. Fig. 13 zeigt eine schematische Schnittdarstellung der dritten Variante für die Einmalvorrichtung 2 in einem Schnitt entlang der Schnittlinie XIII-XIII in Fig. 12, und Fig. 14 zeigt eine schematische Schnittdarstellung der dritten Variante für die Einmalvorrichtung 2 in einem Schnitt entlang der Schnittlinie XIV-XIV in Fig. 12.

Bei dem vierten Ausführungsbeispiel der erfindungsgemässen Pumpvorrichtung 1 ist die wiederverwendbare Vorrichtung 3 - in analoger Weise zu dem dritten Ausführungsbeispiel - derart ausgestaltet, dass die Rotoren 214, 224 der Einmalvorrichtung 2 im Betriebszustand jeweils um die Drehachsen A1, A2 rotieren, wobei die Drehachsen A1 und A2 mit der Vertikalen einen von Null verschieden Winkel einschliessen, der kleiner als 90° ist. Das heisst, die beiden Drehachsen A1 und A2 sind im Betriebszustand unter einem von Null und 90° verschiedenen Winkel gegen die Vertikale geneigt.

Die Einmalvorrichtung 2 ist gemäss einer dritten Variante ausgestaltet, bei welcher der Strom des Fluids in der Einmalvorrichtung 2 in analoger Weise verläuft wie bei der zweiten Variante der Einmalvorrichtung 2. Es ist natürlich auch möglich, dass die dritte Variante der Einmalvorrichtung 2 in sinngemäss gleicher Weise derart ausgestaltet ist, dass die Fluidströmung in analoger Weise wie bei der ersten Variante erfolgt, nämlich derart, dass das Fluid zwischen dem ersten Auslass 212 und dem zweiten Einlass 221 bezüglich der axialen Richtung A lediglich um 90° umgelenkt wird.

Bei der dritten Variante umfasst die Einmalvorrichtung 2 drei Teile 291, 292, 293, die jeweils separat hergestellt werden, und anschliessend in einem Fügeprozess miteinander verbunden werden. Die drei Teile 291, 292, 293 sind so ausgestaltet, dass ihre jeweiligen Grenzflächen senkrecht zur axialen Richtung A ausgerichtet sind. Das erste Teil 291 umfasst die beiden Ausstülpungen 217, 227, mit welchen die Einmalvorrichtung 2 in die wiederverwendbare Vorrichtung 3 einsetzbar ist. Das zweite Teil 292 umfasst denjenigen Bereich, welcher bezüglich der axialen Richtung A an das erste Teil 291 angrenzt, und diejenigen Kavitäten nach oben begrenzt, in welchen die beiden Rotoren 214 und 224 angeordnet sind. Dabei bezieht sich "oben" auf die Darstellung in Fig. 12. Das dritte Teil 293 bildet denjenigen Bereich, welcher die Einmalvorrichtung 2 auf ihrer der wiederverwendbaren Vorrichtung 3 bezüglich der axialen Richtung A abgewandten Seite begrenzt, und umfasst den ersten Einlass 211 sowie Teile des Verbindungskanals 23.

Diese Ausgestaltung ist insbesondere herstellungstechnisch besonders vorteilhaft. Jedes der Teile 291, 292, 293 kann in einfacher Weise, vorzugsweise in einem Spritzgiessverfahren hergestellt werden, und anschliessend werden die drei Teile 291, 292, 293 fest miteinander verbunden.

In den Fig. 13, Fig. 14 sind das erste Teil 291 und das zweite Teil 292 dargestellt. In diesen Schnittdarstellungen erfolgt der Schnitt senkrecht zur axialen Richtung in der Grenzfläche zwischen dem ersten Teil 291 und dem zweiten Teil 292 (Fig. 13) bzw. in der Grenzfläche zwischen dem zweiten Teil 292 und dem dritten Teil 293 (Fig. 14). Die Lage der Schnitte ist in Fig. 12 dargestellt. Fig. 13 zeigt also quasi eine Aufsicht auf den ersten Teil 291 und Fig. 14 eine Aufsicht auf das zweite Teil 292, jeweils aus der axialen Richtung A.

Wie bereits erwähnt ist jedes der drei Teile 291, 292, 293 vorzugsweise ein Spritzgussteil. Zur Herstellung der Einmalvorrichtung 2 wird dabei vorzugsweise wie folgt vorgegangen: Die drei Teile 291, 292, 293 werden in einem Spritzgiessverfahren aus einem Kunststoff gefertigt. Anschliessend wird in die beiden Ausstülpungen 217, 227 des ersten Teils 291 jeweils ein Rotor 214 bzw. 224 eingelegt. Danach wird das zweite Teil 292 auf das erste Teil 291 aufgelegt und in einem Fügeprozess fest und dichtend mit dem ersten Teil 291 verbunden. Dieser Fügeprozess kann beispielsweise ein Verkleben sein, z. B. ein Verkleben mittels eines Klebers, welcher mit ultravioletter Strahlung ausgehärtet werden kann. Ferner kann der Fügeprozess ein Schweissprozess sein, z. B. Infrarotschweissen oder Laserschweissen oder Ultraschallschweissen. Das dritte Teil 293 wird in sinngemäss gleicher Weise mit dem zweiten Teil 292 verbunden. Bei dieser Variante werden somit der Verbindungskanal 23 sowie die Pumpengehäuse 213, 223 jeweils durch Kavitäten gebildet, welche in den drei Teilen 291, 292, 293 vorgesehen sind. Natürlich ist es auch möglich, zunächst alle Teile 291, 292, 293 aufeinander zu legen und erst anschliessend die drei Teile 291, 292, 293 mit einem Schweiss- oder Klebeprozess fest miteinander zu verbinden.

Wie dies insbesondere in Fig. 12 zu erkennen ist, können in der Einmalvorrichtung 2 ein Messkanal 7 oder auch mehrere Messkanäle vorgesehen sein, die sich von einer Aussenseite der Einmalvorrichtung 2 bis in einen der beiden Einlässe 211, 221 oder bis zu einem der Auslässe 212, 222 erstrecken. In einem solchen Messkanal 7 kann ein Sensor (nicht dargestellt) angeordnet werden, mit welchem ein Betriebsparameter, z.B. ein Druck oder der Fluss durch die Pumpvorrichtung 1 ermittelbar ist.

Bei dem vierten Ausführungsbeispiel der erfindungsgemässen Pumpvorrichtung 1 ist ferner in dem Statorgehäuse 35 eine übergeordnete Kontrolleinheit 40 (Fig. 10) vorgesehen. Eine solche übergeordnete Kontrolleinheit 40 kann natürlich auch in den vorangehend beschriebenen Ausführungsbeispielen vorgesehen sein.

Diese übergeordnete Kontrolleinheit 40 ist über eine erste Verbindung 401 mit der ersten Kontrolleinrichtung 41 signalverbunden und über eine zweite Signalverbindung 402 mit der zweiten Kontrolleinrichtung 42 signalverbunden, sodass die übergeordnete Kontrolleinheit 40 mit beiden Kontrolleinrichtungen 41, 42 Signale bzw. Informationen austauschen kann.

Ferner umfasst die Einmalvorrichtung 2 ein Identifizierungselement 81, mit welchem sich die Einmalvorrichtung 2 an der wiederverwendbaren Vorrichtung 3 identifizieren kann. Das Identifizierungselement 81 enthält insbesondere für die Einmalvorrichtung 2 spezifische Daten, beispielsweise Kalibrierungsdaten, sodass die Einmalvorrichtung 2 von der wiederverwendbaren Vorrichtung 3 identifizierbar ist und spezifische Eigenschaften der jeweiligen Einmalvorrichtung 2 an die wiederverwendbare Vorrichtung 3 übermittelt werden können.

Dazu umfasst vorzugsweise die wiederverwendbare Vorrichtung 3 ein Erkennungselement 82, welches derart ausgestaltet ist, dass es über eine Wechselwirkung mit dem Identifizierungselement 81 eine Identifizierung der jeweiligen Einmalvorrichtung 2 bzw. deren Eigenschaften ermöglicht.

Vorzugsweise ist sowohl das Identifizierungselement 81 als auch das Erkennungselement 82 jeweils als RFID (radio-frequency identification) Element ausgestaltet. In anderen Ausgestaltungen kann das Identifizierungselement 81 und/oder das Erkennungselement 82 einen Barcode, insbesondere einen zweidimensionalen oder einen dreidimensionalen Barcode umfassen.

Das Erkennungselement 82 ist über eine Identifizierungsverbindung 801 mit der übergeordneten Kontrolleinheit 40 signalverbunden, sodass die übergeordnete Kontrolleinheit 40 jeweils spezifische Daten, beispielsweise Kalibrierungsdaten, von der in die wiederverwendbare Vorrichtung 3 eingesetzten Einmalvorrichtung 2 empfangen kann und/oder Daten an diese Einmalvorrichtung 2 übermitteln kann.

Ferner sind zwei Drucksensoren 621 und 622 vorgehen, wobei der erste Drucksensor 621 so angeordnet ist, dass mit ihm der Druck des Fluids am ersten Einlass 211 ermittelbar ist, und der zweite Drucksensor 622 so angeordnet ist, dass mit ihm der Druck am zweiten Auslass 222 ermittelbar ist. Mittels der beiden Drucksensoren 621 und 622 lässt sich somit die von der Pumpvorrichtung 1 generierte Druckdifferenz ermitteln.

Die beiden Drucksensoren 621, 622 sind über Verbindungen 611 und 612 mit der übergeordneten Kontrolleinheit 40 signalverbunden. Zusätzlich oder falls keine übergeordnete Kontrolleinheit 40 vorgesehen ist, können die Drucksensoren 621 und 622 mit jeder der Kontrolleinrichtungen 41 und 42 signalverbunden sein. Dies ist in Fig. 10 nur für den Drucksensor 622 am zweiten Auslass 222 mit den gestrichelt dargestellten Verbindungen 623 und 624 dargestellt.

Das dritte Teil 293 kann an seinem dem zweiten Teil 292 zugewandten Ende einen Vorsprung 294 oder mehrere Vorsprünge 294, beispielsweise an jeder Ecke einen, aufweisen, welche zum Zusammenwirken mit Halteelementen 394 auf der Oberfläche des Statorgehäuses 35 ausgestaltet sind (siehe Fig. 11). Vorzugsweise wirken die Vorsprünge 294 an der Einmalvorrichtung 2 mit den Halteelementen 394 an der wiederverwendbaren Vorrichtung 3 in Form von Schnappverbindungen zusammen, welche ein unbeabsichtigtes Trennen der Einmalvorrichtung 2 und der wiederverwendbaren Vorrichtung 3 vermeiden.

Im Folgenden wird nun näher auf das erfindungsgemässe Verfahren zum Betreiben einer erfindungsgemässen Pumpvorrichtung 1 eingegangen. Dabei kann die erfindungsgemässe Pumpvorrichtung 1 gemäss irgendeinem der vorangehenden Ausführungsbeispiele oder Varianten ausgestaltet sein.

Bei dem erfindungsgemässen Verfahren wird ein Soll-Wert für eine Betriebsgrösse der Pumpvorrichtung 1 an jede Kontrolleinrichtung 41, 42 vorgegeben. Mittels eines Sensors wird ein Ist-Wert für diese Betriebsgrösse ermittelt und der Ist-Wert wird an jede Kontrolleinrichtung 41, 42 übermittelt.

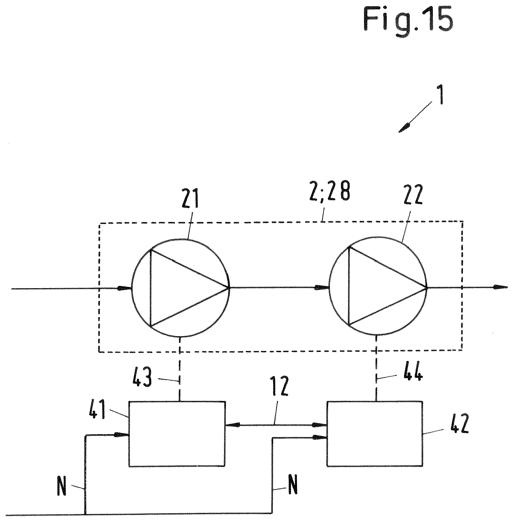

Fig. 15 zeigt in einer symbolischen Darstellung eine Ausgestaltung der erfindungsgemässen Pumpvorrichtung 1 mit den beiden Pumpeneinheiten 21, 22, mit den beiden Kontrolleinrichtungen 41, 42 sowie mit der Kommunikationsverbindung 12 zwischen den beiden Kontrolleinrichtungen 41, 42. Ferner sind symbolisch in gestrichelter Darstellung eine erste Verbindung 43 und eine zweite Verbindung 44 dargestellt. Die erste Verbindung 43 stellt die Kommunikation der ersten Kontrolleinrichtung 41 mit der ersten Pumpeneinheit 21 dar, und die zweite Verbindung stellt die Kommunikation der zweiten Kontrolleinrichtung 42 mit der zweiten Pumpeneinheit 22 dar.

Grundsätzlich basiert das erfindungsgemässe Verfahren darauf, dass für eine vorgegebenen Druckdifferenz, die von der Pumpvorrichtung 1 zu generieren ist, im normalen, das heisst störungsfreien Betrieb die beiden in Serie geschalteten Pumpeneinheit 21, 22 verwendet werden. Hierdurch kann die Drehzahl für jede der beiden Pumpeneinheiten 21, 22 um einen Faktor ($\frac{1}{\sqrt{2}}$) gesenkt werden - im Vergleich zu der Situation, wenn nur eine Pumpeneinheit 21 oder 22 zum Generieren der gleichen Druckdifferenz verwendet wird. Diese Reduzierung der Drehzahl resultiert in einer erheblichen Reduzierung der Scherkräfte, die in den Pumpeneinheiten 21, 22 auf das zu fördernde Fluid einwirken. Dies ist insbesondere beim Fördern von biologischen Substanzen, welche Zellen oder andere empfindliche Substanzen umfassen, also beispielsweise Blut, ein ganz enormer Vorteil.

Falls nun aus welchem Grunde auch immer eine der beiden Pumpeneinheiten 21, 22 bzw. der Kontrolleinrichtungen 41, 42 ausfällt, so wird die Drehzahl der anderen Pumpeneinheit 22 oder 21 um einen Faktor $\sqrt{2}$ erhöht, sodass die gewünschte Druckdifferenz nun nur noch von der einen verbleibenden Pumpeneinheit 22 oder 21.generiert wird. Diese Möglichkeit der heiss redundanten Ausgestaltung ist vor allem für medizinische Anwendungen, z.B das Pumpen von Blut, oder für biotechnologische Anwendungen, z.B. das Züchten von Zellen, ein sehr grosser Vorteil.

In Fig. 15 stellen die Pfeile mit dem Bezugszeichen N den Soll-Wert für die Drehzahl der Pumpeneinheiten 21, 22 dar. Dieser Sollwert N wird an beide Kontrolleinrichtungen 41, 42 übermittelt. Jede Kontrolleinrichtung 41, 42 erhält über die Verbindung 43 bzw. 44 mit der ihr jeweils zugeordneten Pumpeneinheit 21 bzw. 22 den Ist-Wert für die aktuelle Motordrehzahl, mit welcher der jeweilige elektromagnetische Drehantrieb rotiert. Diese Drehzahl wird in einem lagerlosen Motor beispielsweise mittels Hall-Sensoren ermittelt.

Die Pfeile ohne Bezugzeichen an den Pumpeneinheiten 21, 22 symbolisieren das zu fördernde Fluid.

Über die Kommunikationsverbindung 12 können die beiden Kontrolleinrichtungen 41, 42 Kontrollsignale miteinander austauschen und insbesondere auch die jeweiligen Ist-Werte für die Motordrehzahl austauschen. Optional (in Fig. 15 nicht dargestellt) können zusätzliche Signalverbindungen vorgesehen sein, über welche die erste Kontrolleinrichtung 41 direkt - also nicht über die zweite Kontrolleinrichtung 42 - den Ist-Wert der Motordrehzahl der zweiten Pumpeneinheit 22 erhält, und die zweite Kontrolleinrichtung 42 direkt - also nicht über die erste Kontrolleinrichtung 41 - den Ist-Wert der Motordrehzahl der ersten Pumpeneinheit 21 erhält.

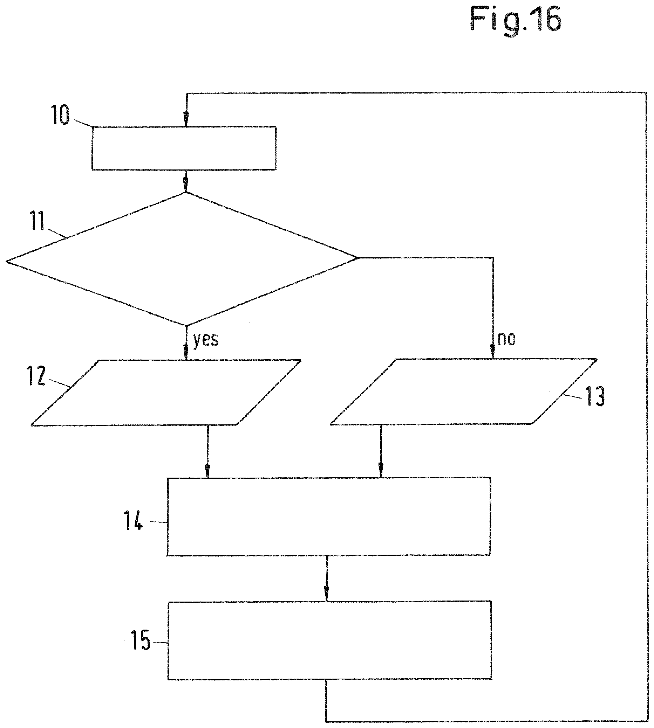

Die vorangehend beschriebene grundlegende Ausführungsform ist in dem Flussdiagramm in Fig. 16 veranschaulicht. Im Schritt 10 liest jede Kontrolleinrichtung 41, 42 den Soll-Wert für die Pumpendrehzahl ein, d.h. den Soll-Wert für die Drehzahl der Pumpvorrichtung 1.

Im Schritt 11 kommunizieren die beiden Kontrolleinrichtungen 41 und 42 über die Kommunikationsverbindung 12 miteinander, um festzustellen, ob der lagerlose Motor der jeweils anderen Pumpeneinheit 21 bzw. 22 fehlerfrei arbeitet. Für solche Überprüfungen sind dem Fachmann zahlreiche Fehlererkennungsprozeduren bekannt.

Wird im Schritt 11 festgestellt, dass die lagerlosen Motoren beider Pumpeneinheiten 21, 22 fehlerfrei arbeiten, so wird im Schritt 12 der Ist-Wert für die Motordrehzahl für beide Pumpeneinheiten 21, 22 jeweils auf einen Wert gesetzt, der um einen Faktor ($\frac{1}{\sqrt{2}}$) kleiner ist als der Soll-Wert für die Pumpendrehzahl.

Wird im Schritt 11 festgestellt, dass einer der beiden lagerlosen Motoren der beiden Pumpeneinheiten 21, 22 nicht arbeitet, so wird im Schritt 13 der Soll-Wert für die Motordrehzahl für den anderen lagerlosen Motor auf den Wert für die Pumpendrehzahl gesetzt.

Im Schritt 14 wird dann nach dem Schritt 12 oder nach dem Schritt 13 überprüft, ob die Differenz zwischen dem Ist-Wert und dem Soll-Wert für die Motordrehzahl innerhalb eines vorgebbaren Toleranzbereichs liegt.

Im Schritt 15 tauschen dann die beiden Kontrolleinrichtungen 41 und 42 das Ergebnis der Überprüfung im Schritt 14 über die Kommunikationsverbindung 12 miteinander aus.

Der Vorgang beginnt dann wieder bei Schritt 10.

Um eine besonders hohe Betriebssicherheit zu gewährleisten, sind vorzugsweise alle Verbindungen, über die Informationen ausgetauscht werden, also beispielsweise die Kommunikationsverbindung 12 redundant ausgestaltet.

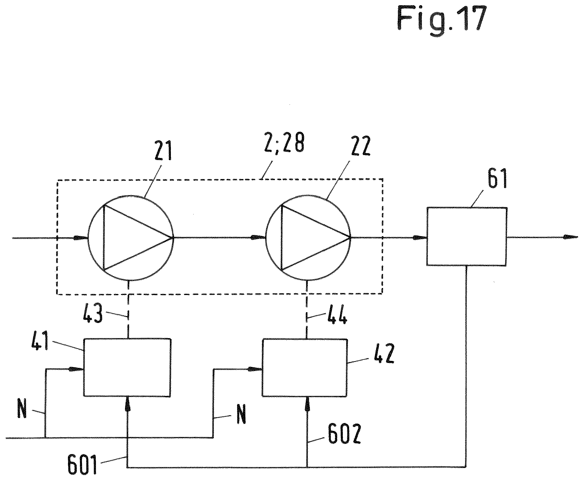

Fig. 17 zeigt eine erste Variante für die Ausführungsform des erfindungsgemässen Verfahrens in einer zu Fig. 15 analogen symbolischen Darstellung. Bei dieser ersten Variante wird als Betriebsgrösse der Fluss durch die Pumpvorrichtung 1 verwendet. Dazu ist der Flusssensor 61 vorgesehen, der vorzugsweise in der Nähe des zweiten Auslasses 222 angeordnet ist. Wie weiter vorne bereits erwähnt, ist der Flusssensor 61 mittels der ersten Signalverbindung 601 mit der ersten Kontrolleinrichtung 41 signalverbunden und mittels der zweiten Signalverbindung 602 mit der zweiten Kontrolleinrichtung 42 signalverbunden. Somit kann der Flusssensor 61 den Ist-Wert des Flusses an die beiden Kontrolleinrichtungen 41, 42 leiten, welche diesen Ist-Wert dann mit dem Soll-Wert für den Fluss vergleichen und bei Abweichungen den Ist-Wert des Flusses auf den Soll-Wert einregeln. Für diese Regelung ist die Kommunikationsverbindung 12 zwischen den beiden Kontrolleinrichtungen 41, 42 nicht unbedingt notwendig. Falls nämlich eine der Kontrolleinrichtungen 41 oder 42 bzw. eine der Pumpeneinheiten 21 oder 22 ausfällt, so führt dies zu einer Änderung des Ist-Wertes für den Fluss, die aufgrund der Signalverbindungen 601, 602 beiden Kontrolleinrichtungen 41, 42 mitgeteilt wird. Die noch funktionierende Kontrolleinrichtung 41 oder 42 bzw. die Kontrolleinrichtung 41, 42 der noch funktionstüchtigen Pumpeneinheit 21 oder 22 wird dann die Drehzahl des zugeordneten lagerlosen Motors derart ändern, dass der Ist-Wert des Flusses wieder auf den Soll-Wert des Flusses geführt wird.

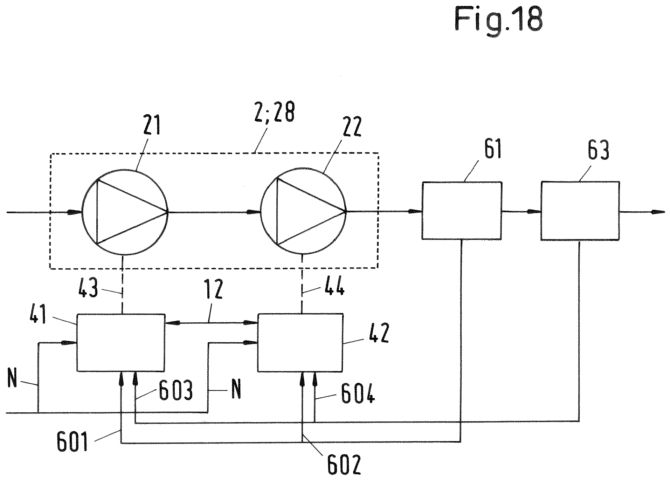

Fig. 18 zeigt eine zweite Variante für die Ausführungsform des erfindungsgemässen Verfahrens in einer zu Fig. 15 analogen symbolischen Darstellung. Bei dieser zweiten Variante wird als Betriebsgrösse ebenfalls der Fluss durch die Pumpvorrichtung 1 verwendet. Im Unterschied zu der ersten Variante ist bei der zweiten Variante die Flussmessung zur Ermittlung des Ist-Wertes des Flusses redundant ausgestaltet. Dies kann beispielsweise wie in Fig. 18 gezeigt derart realisiert werden, dass zusätzlich zu dem Flusssensor 61 ein zweiter Flusssensor 63 vorgesehen ist. Jeder Flusssensor 61, 63 ist dann mit jeder Kontrolleinrichtung 41, 42 signalverbunden Der Flusssensor 61 ist über die Signalverbindungen 601, 602 mit der ersten Kontrolleinrichtung 41 bzw. mit der zweiten Kontrolleinrichtung 42 signalverbunden, und der zweite Flusssensor 63 ist über Signalverbindungen 603, bzw. 604 mit der ersten Kontrolleinrichtung 41 bzw. mit der zweiten Kontrolleinrichtung 42 signalverbunden. Alternativ ist es natürlich auch möglich, anstelle von zwei Flusssensoren 61, 63 nur einen Flusssensor vorzusehen, welcher derart redundant ausgestaltet ist, beispielsweise über die Anzahl der Ultraschallwandler, dass er zwei voneinander unabhängige Messungen des Flusses ermöglicht.

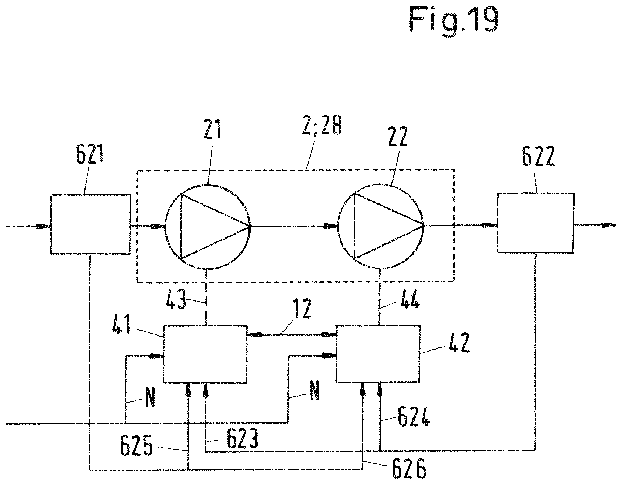

Fig. 19 zeigt eine dritte Variante für die Ausführungsform des erfindungsgemässen Verfahrens in einer zu Fig. 15 analogen symbolischen Darstellung. Bei dieser dritten Variante wird als Betriebsgrösse die von der Pumpvorrichtung 1 generierte Druckdifferenz verwendet. Hierzu sind zwei Drucksensoren 621 und 622 vorgehen (siehe auch Fig. 10), wobei der erste Drucksensor 621 so angeordnet ist, dass mit ihm der Druck des Fluids am ersten Einlass 211 ermittelbar ist, und der zweite Drucksensor 622 so angeordnet ist, dass mit ihm der Druck am zweiten Auslass 222 ermittelbar ist. Mittels der beiden Drucksensoren 621 und 622 lässt sich somit die von der Pumpvorrichtung 1 generierte Druckdifferenz ermitteln. Jeder der beiden Drucksensoren 621 und 622 ist jeweils mit beiden Kontrolleinrichtungen 41 und 42 signalverbunden. Der Drucksensor 622 ist mittels der Verbindungen 623 bzw. 624 mit der ersten Kontrolleinrichtung 41 bzw. mit der zweiten Kontrolleinrichtung 42 signalverbunden.

Der Drucksensor 621 ist mittels der Verbindungen 625 bzw. 626 mit der ersten Kontrolleinrichtung 41 bzw. mit der zweiten Kontrolleinrichtung 42 signalverbunden.

Somit können die beide Drucksensoren 621 und 622 den Ist-Wert des Drucks am ersten Einlass 211 und den Ist-Wert des Druckes am zweiten Auslass 222 jeweils an beide Kontrolleinrichtungen 41, 42 übermitteln. Die Kontrolleinrichtungen 41, 42 bestimmen dann jeweils den Ist-Wert für die von der Pumpvorrichtung 1 generierte Druckdifferenz und vergleichen diesen mit dem Soll-Wert für die Druckdifferenz. Bei Abweichungen zwischen dem Ist-Wert und dem Soll-Wert für die Druckdifferenz, welche einen vorgebbaren Toleranzbereich überschreiten, wird der Ist-Wert der Druckdifferenz auf den Soll-Wert eingeregelt. Für diese Regelung ist die Kommunikationsverbindung 12 zwischen den beiden Kontrolleinrichtungen 41, 42 nicht unbedingt notwendig. Falls nämlich eine der Kontrolleinrichtungen 41 oder 42 bzw. eine der Pumpeneinheiten 21 oder 22 ausfällt, so führt dies zu einer Änderung des Ist-Wertes für die Druckdifferenz, die von beiden Kontrolleinrichtungen 41, 42 detektierbar ist. Die noch funktionierende Kontrolleinrichtung 41 oder 42 bzw. die Kontrolleinrichtung 41, 42 der noch funktionstüchtigen Pumpeneinheit 21 oder 22 wird dann die Drehzahl des zugeordneten lagerlosen Motors derart ändern, dass der Ist-Wert der Druckdifferenz wieder auf den Soll-Wert der Druckdifferenz geführt wird.

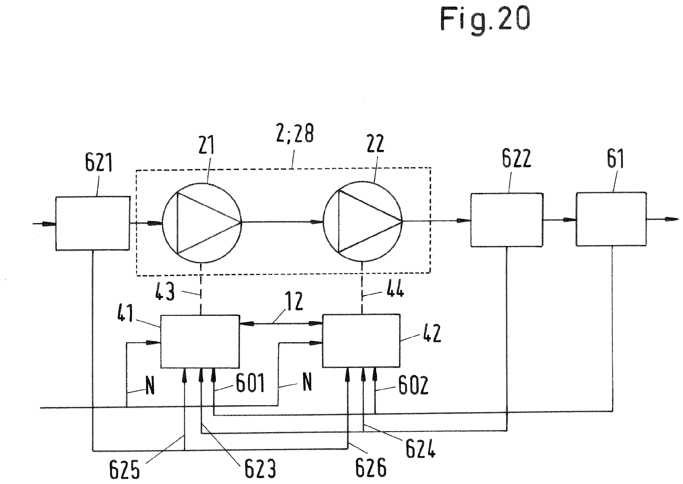

Fig. 20 zeigt eine vierte Variante für die Ausführungsform des erfindungsgemässen Verfahrens in einer zu Fig. 15 analogen symbolischen Darstellung. Bei dieser vierten Variante werden zwei Betriebsgrössen verwendet, nämlich die von der Pumpvorrichtung 1 generierte Druckdifferenz und der Fluss durch die Pumpvorrichtung 1. Diese vierte Variante ist somit eine Kombination der dritten Variante (Fig. 19) mit der zweiten Variante (Fig. 18) oder mit der ersten Variante (Fig. 17). Bei der vierten Variante sind also sowohl die Drucksensoren 621 und 622 vorgesehen, anhand derer die von der Pumpvorrichtung 1 generierte Druckdifferenz ermittelbar ist, als auch der Flusssensor 61, mit welchem der Fluss durch die Pumpvorrichtung 1 ermittelt werden kann. Dabei ist es sowohl möglich, dass nur ein Flusssensor 61 vorgesehen ist, wie anhand von Fig. 17 erläutert, oder dass zwei Flusssensoren 61, 63 (siehe Fig. 18) vorgesehen sind bzw. ein redundant ausgestalteter Flusssensor 61.

Insbesondere im Hinblick auf die Betriebsgrössen Druckdifferenz und Fluss ist es auch möglich, dass diese Betriebsgrössen alternativ oder ergänzend aus anderen Betriebsgrössen der Pumpvorrichtung 1 ermittelt werden.

So kann man beispielsweise aus der Drehzahl des lagerlosen Motors und dem generierten Drehmoment den Fluss durch die Pumpvorrichtung 1 ermitteln. Das Drehmoment ist bei einem lagerlosen Motor sehr genau aus den elektrischen Grössen bekannt, weil beim lagerlosen Motor keine Reibung wie beispielsweise bei mechanischen Lagern auftritt.

Man kann dann das Drehmoment für verschiedene Drehzahlen als eine Funktion darstellen, die von der Dichte des Fluids, von seiner Viskosität (dynamische Viskosität) und vom Fluss abhängt. Mit einer Referenzflüssigkeit, beispielsweise menschliches oder tierisches Blut bei einer Temperatur von 37°C kann dann experimentell für verschiedene Drehzahlen ein Kennlinienfeld aufgenommen werden, welches die Abhängigkeit des Flusses vom Drehmoment wiedergibt. Dieses Kennlinienfeld kann dann wie bereits erwähnt durch eine Funktionenschar abgebildet werden, um so die Koeffizienten für die Parameter in dem funktionalen Zusammenhang zu ermitteln. Diese Parameter bzw. der funktionale Zusammenhang können dann in den Kontrolleinrichtungen 41, 42 hinterlegt werden, sodass während des Betriebs aus dem Ist-Wert des Drehmoments der Ist-Wert des Flusses ermittelt werden kann. Alternativ ist es natürlich auch möglich, das gesamte Kennlinienfeld, beispielsweise als Look-up Tabelle in den beiden Kontrolleinrichtungen 41, 42 zu hinterlegen.

Je nachdem, welche Grössen aus dem Kennlinienfeld Drehmoment-Fluss bekannt sind, ist es auch möglich, andere Grössen des Fluids zu ermitteln. In dem Drehmoment-Fluss-Kennlinienfeld ist beispielsweise das Drehmoment beim Fluss Null proportional zur Viskosität. Wenn also beispielsweise der Fluss messtechnisch bestimmt wird, so kann die Viskosität des Fluids ermittelt werden. So lässt sich beispielsweise auch ein Drehmoment-Fluss-Kennlinienfeld generieren, bei welchem die Viskosität der Parameter ist, das heisst, die unterschiedlichen Kurven des Kennlinienfeldes gehören zu unterschiedlichen Viskositäten.

Die erfindungsgemässe Pumpvorrichtung 1 bzw. das erfindungsgemässe Verfahren eignen sich insbesondere für Anwendungen, bei welchen sehr empfindliche Substanzen, beispielsweise Blut oder biotechnologische Fluide mit Zellen, Proteinen oder anderen empfindlichen Inhaltsstoffen, gefördert werden. Mit beispielhaftem Charakter seien hier genannt: Die extrakorporale Membranoxigenierung (ECMO: extracorporal membran oxigenation) zur Unterstützung der Lungenfunktion, Anwendungen in Herz-Lungen-Maschinen, biopharmazeutische Herstellungsprozesse, beispielsweise Filtrationsprozesse, bei welchem einem Fluid, das in einem Bioreaktor erzeugt wird, die gewünschten Substanzen (beispielsweise Proteine) als Permeat entnommen wird, Perfusionsprozesse in der biotechnologische oder biopharmazeutischen Industrie.

In der vorangehenden Beschreibung sind Pumpvorrichtungen 1 erläutert, welche zwei Pumpeneinheiten 21, 22 aufweisen. Es sind aber auch solche Ausgestaltungen der erfindungsgemässen Pumpvorrichtung möglich, bei denen mehr als zwei Pumpeneinheiten, beispielsweise drei oder noch mehr Pumpeneinheiten in Serie hintereinandergeschaltet sind.

## Patentansprüche

1. Pumpvorrichtung zum Fördern eines Fluids mit einer Einmalvorrichtung (2), die für den Einmalgebrauch ausgestaltet ist, und mit einer wiederverwendbaren Vorrichtung (3), die für den Mehrfachgebrauch ausgestaltet ist, wobei die Einmalvorrichtung (2) zum Einsetzen in die wiederverwendbare Vorrichtung (3) ausgestaltet ist, und zwei Pumpeneinheiten (21, 22) umfasst, welche in Serie hintereinander angeordnet sind, wobei jede Pumpeneinheit (21, 22) einen Rotor (214, 224) zum Fördern des Fluids umfasst, wobei jeder Rotor (214, 224) als Rotor eines lagerlosen Motors ausgestaltet ist, und berührungslos magnetisch lagerbar sowie berührungslos zur Rotation um eine axiale Richtung (A) antreibbar ist, wobei die wiederverwendbare Vorrichtung (3) zum Einsetzen der Einmalvorrichtung (2) ausgestaltet ist, und für jeden Rotor (214, 224) einem Stator (31, 32) umfasst, der mit dem Rotor (214, 224) einen elektromagnetischen Drehantrieb zum Rotieren des Rotors (214, 224) um die axiale Richtung (A) bildet, wobei jeder Stator (31, 32) als Lager- und Antriebsstator ausgestaltet ist, mit welchem der Rotor (214, 224) berührungslos magnetisch antreibbar und berührungslos magnetisch bezüglich des Stators (31, 32) lagerbar ist, und wobei für jeden Stator (31, 32) eine unabhängige Kontrolleinrichtung (41, 42) vorgesehen ist, welche für eine unabhängige Ansteuerung des jeweiligen Stators (31, 32) ausgestaltet ist.

2. Pumpvorrichtung nach Anspruch 1, wobei die Einmalvorrichtung (2) zwei becherförmige Ausstülpungen (217, 227) aufweist, in welchen jeweils ein Rotor (214, 224) vorgesehen ist, und wobei die wiederverwendbare Vorrichtung (3) zwei Ausnehmungen (33, 34) aufweist, von denen jede zur Aufnahme einer der becherförmigen Ausstülpungen (217, 227) ausgestaltet ist.

3. Pumpvorrichtung nach Anspruch 2, wobei die wiederverwendbare Vorrichtung (3) derart ausgestaltet ist, dass die Rotoren (214, 224) der Einmalvorrichtung (2) im Betriebszustand jeweils um eine Drehachse (A1, A2) rotieren, welche mit der Vertikalen einen von Null verschiedene Winkel einschliesst, der kleiner als 90° ist.

4. Pumpvorrichtung nach einem der vorangehenden Ansprüche, wobei die erste Pumpeneinheit (21) einen ersten Einlass (211) sowie einen ersten Auslass (212) für das Fluid aufweist, wobei die zweite Pumpeneinheit (22) einen zweiten Einlass (221) sowie einen zweiten Auslass (222) für das Fluid aufweist, wobei jeder Einlass (211, 221) so ausgestaltet ist, dass das Fluid den jeweiligen Rotor (214, 224) aus einer axialen Richtung (A) anströmt, und wobei jeder Auslass (212, 222) so ausgestaltet ist, dass das Fluid die jeweilige Pumpeneinheit (21, 22) in einer Austrittsrichtung (D) verlässt, welche senkrecht zur axialen Richtung (A) ausgerichtet ist.

5. Pumpvorrichtung nach Anspruch 4, welche derart ausgestaltet ist, dass das Fluid zwischen dem ersten Auslass (212) und dem zweiten Einlass (221) um mindestens 90° umgelenkt wird.

6. Pumpvorrichtung nach einem der Ansprüche 4-5, welche derart ausgestaltet ist, dass das Fluid zwischen dem ersten Auslass (212) und dem zweiten Einlass (221) um insgesamt 270° umgelenkt wird.

7. Pumpvorrichtung nach einem der vorangehenden Ansprüche, wobei für jeden Stator (31, 32) jeweils eine separate Energieversorgung (51, 52) vorgesehen ist, sodass beim Ausfall einer der Energieversorgungen (51, 52) noch einer der Statoren (31, 32) mit Energie versorgbar ist.

8. Pumpvorrichtung nach einen der vorangehenden Ansprüche, wobei eine übergeordnete Kontrolleinheit (40) vorgesehen ist, welche mit allen Kontrolleinrichtungen (41, 42) für die Statoren (31, 32) signalverbunden ist.

9. Pumpvorrichtung nach einem der vorangehenden Ansprüche, wobei ein Notfallenergiespeicher (512, 522) vorgesehen ist, aus welchem jedem Stator (31, 32) Energie zuführbar ist, falls eine Primärenergiequelle (511, 521) zur Versorgung der Statoren (31, 32) einer oder allen Pumpeneinheiten (21, 22) keine Energie mehr zur Verfügung stellen kann.

10. Pumpvorrichtung nach einem der vorangehenden Ansprüche, wobei die Einmalvorrichtung (2) und/oder die wiederverwendbare Vorrichtung (3) ein Identifizierungselement (81, 82) enthalten, mit welchem die Einmalvorrichtung (2) und die wiederverwendbare Vorrichtung (3) Informationen miteinander austauschen können.

11. Einmalvorrichtung, die für den Einmalgebrauch und für eine Pumpvorrichtung (1) ausgestaltet ist, welche nach einem der vorangehenden Ansprüche ausgestaltet ist.

12. Verfahren zum Betreiben einer Pumpvorrichtung, die nach einem der Ansprüche 1-10 ausgestaltet ist, wobei ein Soll-Wert für eine Betriebsgrösse der Pumpvorrichtung (1) an jede Kontrolleinrichtung (41, 42) vorgegeben wird, wobei mittels eines Sensors (6, 61, 63, 621, 622) ein Ist-Wert für diese Betriebsgrösse ermittelt wird, und wobei der Ist-Wert an jede Kontrolleinrichtung (41, 42) übermittelt wird.

13. Verfahren nach Anspruch 12, wobei die Kontrolleinrichtungen (41, 42) Signale miteinander austauschen, mittels welchen jede Kontrolleinrichtung (41, 42) das Funktionieren der anderen Pumpeneinheit (21, 22) ermitteln kann.

14. Verfahren nach einem der Ansprüche 12-13, wobei die Betriebsgrösse der Fluss durch die Pumpvorrichtung (1) oder die von der Pumpvorrichtung (1) generierte Druckdifferenz ist.

15. Verfahren nach einem der Ansprüche 12-14, bei welchen der Fluss durch die Pumpvorrichtung (1) aus der Drehzahl und dem Drehmoment ermittelt wird, mit welchen die Rotoren (214, 224) angetrieben werden.

**Claims**

1. A pumping device for conveying a fluid with a single-use device (2) designed for single use and with a reusable device (3) designed for multiple use, wherein the single-use device (2) is designed to be inserted into the reusable device (3) and comprises two pump units (21, 22) arranged in series one behind the other, wherein each pump unit (21, 22) comprises a rotor (214, 224) for conveying the fluid, wherein each rotor (214, 224) is designed as a rotor of a bearingless motor, and can be magnetically levitated without contact and driven without contact for rotation about an axial direction (A), wherein the reusable device (3) is designed for inserting the single-use device (2) and comprises for each rotor (214, 224) a stator (31, 32) which forms with the rotor (214, 224) an electromagnetic rotary drive for rotating the rotor (214, 224) about the axial direction (A), wherein each stator (31, 32) is designed as a bearing and drive stator with which the rotor (214, 224) can be magnetically driven without contact and can be magnetically levitated without contact with respect to the stator (31, 32), and wherein an independent control device (41, 42) is provided for each stator (31, 32), which is designed for an independent activation of the respective stator (31, 32).

2. A pumping device according to claim 1, wherein the single-use device (2) has two cup-shaped protuberances (217, 227) in each of which a rotor (214, 224) is provided, and wherein the reusable device (3) has two recesses (33, 34), each of which is designed to receive one of the cup-shaped protuberances (217, 227).

3. A pumping device according to claim 2, wherein the reusable device (3) is designed in such a way that, in the operating state, the rotors (214, 224) of the single-use device (2) each rotate about an axis of rotation (A1, A2) which encloses with the vertical an angle different from zero, which is smaller than 90°.

4. A pumping device according to anyone of the preceding claims, wherein the first pump unit (21) has a first inlet (211) and a first outlet (212) for the fluid, wherein the second pump unit (22) has a second inlet (221) and a second outlet (222) for the fluid, wherein each inlet (211, 221) is designed such that the fluid flows to the respective rotor (214, 224) from an axial direction (A), and wherein each outlet (212, 222) ) is designed such that the fluid leaves the respective pump unit (21, 22) in a discharge direction (D) which is aligned perpendicular to the axial direction (A).

5. A pumping device according to claim 4, which is designed in such a way that the fluid is diverted between the first outlet (212) and the second inlet (221) by at least 90°.

6. A pumping device according to anyone of the claims 4 to 5, which is designed in such a way that the fluid is diverted between the first outlet (212) and the second inlet (221) by a total of 270°.

7. A pumping device according to anyone of the preceding claims, wherein a separate power supply (51, 52) is provided for each stator (31, 32) in each case, so that if one of the power supplies (51, 52) fails, one of the stators (31, 32) can still be supplied with power.

8. A pumping device according to anyone of the preceding claims, wherein a superordinate control unit (40) is provided which is signal-connected to all control devices (41, 42) for the stators (31, 32).

9. A pumping device according to anyone of the preceding claims, wherein an emergency energy store (512, 522) is provided from which energy can be supplied to each stator (31, 32) if a primary energy source (511, 521) for supplying the stators (31, 32) can no longer provide energy to one or all pump units (21, 22).

10. A pumping device according to anyone of the preceding claims, wherein the single-use device (2) and/or the reusable device (3) contain an identification element (81, 82) with which the single-use device (2) and the reusable device (3) can exchange information with each another.

11. A single-use device designed for single use and for a pumping device (1) which is designed according to anyone of the preceding claims.

12. A method for operating a pumping device which is designed according to anyone of the claims 1 to 10, wherein a desired value for an operating parameter of the pumping device (1) is predetermined to each control device (41, 42), wherein an actual value for this operating parameter is determined by means of a sensor (6, 61, 63, 621, 622), and wherein the actual value is transmitted to each control device (41, 42).

13. A method according to claim 12, wherein the control devices (41, 42) exchange signals with each other, by means of which each control device (41, 42) can determine the functioning of the other pump unit (21, 22).

14. A method according to anyone of the claims 12 to 13, wherein the operating parameter is the flow through the pumping device (1) or the pressure difference generated by the pumping device (1).

15. A method according to anyone of the claims 12 to 14, in which the flow through the pumping device (1) is determined from the rotational speed and the torque with which the rotors (214, 224) are driven.

**Revendications**

1. Dispositif de pompage pour le convoyage d'un fluide avec un dispositif jetable (2) qui est conçu pour un usage unique, et avec un dispositif réutilisable (3), qui est conçu pour un usage multiple, dans lequel le dispositif jetable (2) est conçu pour être inséré dans le dispositif réutilisable (3), et qui comprend deux unités de pompage (21, 22), dans lequel chaque unité de pompage (21, 22) comprend un rotor (214, 224) pour le convoyage du fluide, dans lequel chaque rotor (214, 224) est conçu comme un rotor d'un moteur sans palier et peut être logé magnétiquement sans contact et entraîné sans contact pour une rotation autour d'une direction axiale (A), dans lequel le dispositif réutilisable (3) est conçu pour l'insertion du dispositif jetable (2) et, pour chaque rotor (214, 224), comprend un stator (31, 32), qui constitue, avec le rotor (214, 224), un entraînement rotatif pour la rotation du rotor (214, 224) autour de la direction axiale (A), dans lequel chaque stator (31, 32) est conçu comme un stator de palier et d'entraînement avec lequel le rotor (214, 224) peut être entraîné magnétiquement sans contact et peut être logé en relation avec le stator (31, 32), et dans lequel, pour chaque stator (31, 32), un dispositif de contrôle indépendant (41, 42) est prévu, qui est conçu pour un contrôle indépendant du stator (31, 32) respectif.

2. Dispositif de pompage selon la revendication 1, dans lequel le dispositif jetable (2) comprend deux saillies (217, 227) en forme de godet, dans chacun desquels est disposé un rotor (214, 224) et dans lequel le dispositif réutilisable (3) comprend deux évidements (33, 34), dont chacun est conçu pour le logement d'une des saillies (217, 227) en forme de godet.

3. Dispositif de pompage selon la revendication 2, dans lequel le dispositif réutilisable (3) est conçu de sorte que les rotors (214, 224) du dispositif jetable (2) tournent respectivement, dans l'état de fonctionnement, autour d'un axe de rotation (A1, A2) qui forme, avec la verticale, un angle différent de zéro, qui est inférieur à 90°.

4. Dispositif de pompage selon l'une des revendications précédentes, dans lequel la première unité de pompage (21) comprend une première entrée (211) ainsi qu'une première sortie (212) pour le fluide, dans lequel la deuxième unité de pompage (22) comprend une deuxième entrée (221) ainsi qu'une deuxième sortie (222) pour le fluide, dans lequel chaque entrée (211, 221) est conçue de sorte que le fluide entre dans le rotor (214, 224) respectif à partir d'une direction axiale (A) et dans lequel chaque sortie (212, 222) est conçue de sorte que le fluide quitte l'unité de pompage (21, 22) respective dans une direction de sortie (D) qui est orientée perpendiculairement à la direction axiale (A).

5. Dispositif de pompage selon la revendication 4, qui est conçu de sorte que le fluide est dévié, entre la première sortie (212) et la deuxième entrée (221), d'au moins 90°.

6. Dispositif de pompage selon l'une des revendications 4 - 5, qui est conçu de sorte que le fluide est dévié, entre la première sortie (212) et la deuxième entrée (221), de 270° au total.

7. Dispositif de pompage selon l'une des revendications précédentes, dans lequel, pour chaque stator (31, 32), est prévue une alimentation en énergie (51, 52) séparée, de sorte que, lors d'une panne d'une des alimentations en énergie (51, 52), un des stators (31, 32) peut encore être alimenté en énergie.

8. Dispositif de pompage selon l'une des revendications précédentes, dans lequel une unité de contrôle prioritaire (40) est prévue, qui est reliée par signaux avec toutes les unités de contrôle (41, 42) pour les stators (31, 32).

9. Dispositif de pompage selon l'une des revendications précédentes, dans lequel un accumulateur d'énergie de secours (512, 522) est prévu, à partir duquel chaque stator (31, 32) peut être alimenté en énergie dans le cas où une source d'énergie primaire (511, 521) pour l'alimentation des stators (31, 32) d'une ou de toutes les unités de pompage (21, 22) ne peut pas fournir de l'énergie.

10. Dispositif de pompage selon l'une des revendications précédentes, dans lequel le dispositif jetable (2) et/ou le dispositif réutilisable (3) contiennent un élément d'identification (81, 82) avec lequel le dispositif jetable (2) et le dispositif réutilisable (3) peuvent échanger des informations entre eux.

11. Dispositif jetable qui est conçu pour un usage unique et pour un dispositif de pompage (1) qui est conçu selon l'une des revendications précédentes.

12. Procédé de fonctionnement d'un dispositif de pompage qui est conçu selon l'une des revendications 1 - 10, dans lequel une valeur de consigne pour une grandeur de fonctionnement du dispositif de pompage (1) est prédéterminée au niveau de chaque dispositif de contrôle (41, 42), dans lequel, au moyen d'un capteur (6, 61, 63, 621, 622), une valeur réelle pour cette grandeur de fonctionnement est déterminée, et dans lequel la valeur réelle est transmise à chaque dispositif de contrôle (41, 42).

13. Procédé selon la revendication 12, dans lequel les dispositifs de contrôle (41, 42) échangent entre eux des signaux au moyen desquels chaque dispositif de contrôle (41, 42) peut déterminer le fonctionnement de l'autre unité de pompage (21, 22).

14. Procédé selon l'une des revendications 12 à 13, dans lequel la grandeur de fonctionnement est le débit à travers le dispositif de pompage (1) ou la différence de pression générée par le dispositif de pompage (1).

15. Procédé selon l'une des revendications 12 à 14, dans lequel le débit à travers le dispositif de pompage (1) est déterminé à partir de la vitesse de rotation et du couple avec lesquels les rotors (214, 224) sont entraînés.

Fig.1

Fig.2

Fig.3

Fig.4

Fig.5

Fig.6

Fig.7

Fig.8

Fig.9

Fig.10

Fig.11

Fig.12

Fig.13

Fig.14

Fig.15

Fig.16

# Fig.17

Fig.18

Fig.19

Fig.20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0860046 A
- EP 0819330 A

- US 2017040868 A